(19) Europäisches Patentamt
European Patent Office
Office européen des brevets

(11) EP 1 134 217 A2

(12) **DEMANDE DE BREVET EUROPEEN**

(43) Date de publication:
**19.09.2001 Bulletin 2001/38**

(21) Numéro de dépôt: **01400498.0**

(22) Date de dépôt: **27.02.2001**

(51) Int Cl.[7]: **C07D 307/92**, C07D 493/04, C07D 491/04, A61K 7/13, A61K 7/021, A61K 7/032 // (C07D493/04, 307:00, 307:00), (C07D491/04, 307:00, 221:00)

(84) Etats contractants désignés:
**AT BE CH CY DE DK ES FI FR GB GR IE IT LI LU MC NL PT SE TR**
Etats d'extension désignés:
**AL LT LV MK RO SI**

(30) Priorité: **17.03.2000 FR 0003471**

(71) Demandeur: **L'OREAL**
**75008 Paris (FR)**

(72) Inventeurs:
- **Henrion, Jean-Christophe**
**93500 Pantin (FR)**
- **Philippe, Michel**
**91320 Wissous (FR)**

(74) Mandataire: **Le Blainvaux Bellegarde, Françoise**
**L'OREAL-DPI**
**6 rue Bertrand Sincholle**
**92585 Clichy Cedex (FR)**

(54) **Composition cosmétique comprenant un dérivé de furane-naphtoquinone, leur utilisation comme agent colorant, et dérivés.**

(57) La présente demande concerne une composition cosmétique comprenant des dérivés de furane-naphtoquinone de formule (I) :

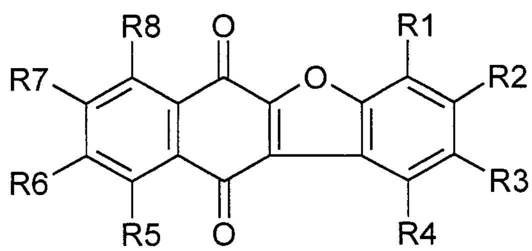

L'invention a également pour objet l'utilisation d'au moins un composé de formule (I) tel que défini ci-dessus en tant qu'agent colorant, notamment dans une composition cosmétique, ainsi que certains nouveaux dérivés de furane-naphtoquinone.

EP 1 134 217 A2

## Description

La présente invention a trait à l'utilisation en cosmétique de composés dérivés de furane-naphtoquinones, notamment dans des compositions cosmétiques.

Les compositions cosmétiques, et notamment les compositions de maquillage telles que les poudres libres ou compactes, les fonds de teint, les fards à joues ou à paupières, les rouges à lèvres ou les vernis à ongles, sont constituées d'un véhicule approprié et de différents agents colorants destinés à conférer une certaine couleur auxdites compositions avant et/ou après leur application sur la peau, les muqueuses et/ou les phanères telles que les ongles, les cils ou les cheveux.

Pour créer des couleurs, on utilise aujourd'hui une gamme d'agents colorants assez limitée, parmi lesquels on peut citer des composés généralement insolubles dans les milieux aqueux et organiques tels que des laques organiques, des pigments minéraux ou des pigments nacrés.

Les pigments et laques utilisés dans le domaine du maquillage sont d'origine et de nature chimique très diverses. Leurs propriétés physico-chimiques, notamment granulométrie, surface spécifique, densité, etc., sont donc très différentes. Ces différences se traduisent par des variations de comportement : leur facilité de mise en oeuvre, de dispersion dans le milieu; leur stabilité à la lumière, à la température; leurs propriétés mécaniques.

Ainsi, les pigments minéraux, en particulier les oxydes minéraux tels que les oxydes de fer, sont très stables à la lumière et au pH, mais donnent des couleurs plutôt ternes, fades et pâles. Il est donc nécessaire d'en introduire une grande quantité dans les formulations cosmétiques pour obtenir un trait suffisamment saturé. Ce fort pourcentage de particules minérales peut néanmoins affecter la brillance de la composition.

Pour obtenir des effets colorés, on peut encore employer des pigments nacrés de couleurs variées, mais jamais très intenses, qui permettent d'obtenir des effets irisés mais le plus souvent assez faibles.

Dans le domaine de la coloration capillaire temporaire ou fugace, qui donne lieu à une modification légère de la couleur naturelle de la chevelure qui tient d'un shampooing à l'autre et qui sert à embellir ou corriger une nuance déjà obtenue, on a déjà proposé une coloration avec des pigments minéraux usuels pour apporter un reflet temporaire aux cheveux, mais les nuances obtenues par cette coloration restent assez ternes, trop uniformes et peu ludiques.

Dans le domaine du maquillage, seules les laques organiques permettaient jusqu'à présent d'obtenir des couleurs vives et intenses. Cependant, la plupart des laques organiques présentent une très mauvaise tenue à la lumière, qui se traduit par une atténuation très nette de leur couleur dans le temps. Elles peuvent également être instables à la température et/ou au pH. De plus, certaines laques génèrent un dégorgement trop important, c'est-à-dire qu'elles présentent l'inconvénient de tacher le support sur lequel elles sont appliquées. Ainsi, ceci peut avoir pour conséquence de tâcher les lentilles oculaires dans le cas des eye-liners ou des mascaras, ou de laisser une coloration sur la peau ou les ongles après démaquillage dans le cas des rouges à lèvres ou des vernis à ongles. Enfin, l'instabilité des laques est encore aggravée lorsqu'elles sont associées à des pigments photoréactifs comme le dioxyde de titane. Or ces pigments sont très largement utilisés dans le maquillage, notamment pour la protection contre le rayonnement UV. Par conséquent, l'utilisation des laques organiques en cosmétique est assez limitée, ce qui a pour conséquence une limitation des teintes réalisables.

Ainsi il subsiste le besoin de disposer d'agents colorants susceptibles d'être utilisés en cosmétique, permettant d'obtenir une coloration adéquate des compositions et du maquillage obtenu, lesdits agents colorants ayant par ailleurs une bonne couvrance, une bonne stabilité thermique et photochimique, tout en présentant un faible dégorgement.

Après de nombreuses recherches, la demanderesse a mis en évidence que l'utilisation d'une famille bien précise de composés, dérivés de furane-naphtoquinones, permettait d'obtenir de manière inattendue, un tel résultat.

Ainsi l'invention a pour objet une composition cosmétique comprenant, dans un milieu cosmétiquement acceptable, au moins un composé de formule (I) :

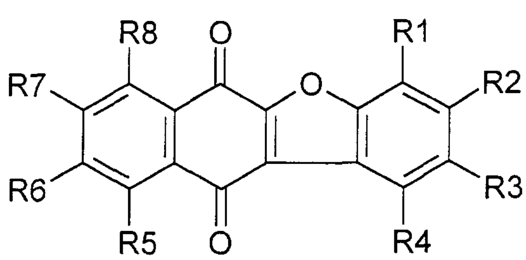

dans laquelle les radicaux R1 à R8 sont tels que définis ci-après.

L'invention a également pour objet l'utilisation d'au moins un composé de formule (I) tel que défini ci-dessous en tant qu'agent colorant, notamment dans une composition cosmétique.

Les composés dérivés de furane-naphtoquinones employés dans le cadre de la présente invention sont, pour certains d'entre eux, des composés connus dans la littérature.

Ainsi, certains ont notamment été décrits dans la publication de Shand et al., Tetrahedron, 1963, vol.19, pp1919-1937 qui traite de la formation de dérivés de dibenzofurannes par réarrangement de diquinones.

D'autres sont décrits dans la publication de Sankaram et al., Phytochemistry, 1981, vol.20, n°5, pp.1093-1096 qui traite des quinones pentacycliques extraites du bois de *Diospyros Melanoxylon.*

D'autres sont également décrits dans la publication en japonais de Ishikawa et al, Nihon Kagakkai-shi, 1988, n°5, pp.743-751, qui traite de la synthèse de composés du type dibenzofurannes ou dinaphtofuranequinone.

Aucune de ces publications ne laisse toutefois envisager que ces composés peuvent être employés avec succès en tant que pigment dans des compositions cosmétiques. Il revient à la demanderesse le mérite d'avoir constaté qu'une telle utilisation était possible.

Parmi les avantages que peuvent procurer ces composés, on peut noter leur bonne stabilité à la température, au pH et à la lumière.

Ils se présentent sous forme solide et produisent des couleurs vives et variées, selon la nature des substituants.

Leur couleur est pure et très saturée, et s'étale dans une gamme très large, allant du jaune au rouge foncé.

Leur force colorante (ou perte en saturation) et leur couvrance sont également très bonnes et comparables à celles des pigments de l'état de la technique.

De plus, ces composés sont généralement insolubles dans l'eau et très peu solubles dans des huiles de nature et /ou de polarité variées. En conséquence, ces composés présentent l'avantage de très peu dégorger lorsqu'ils sont utilisés dans des compositions comprenant des corps gras.

On a également constaté qu'il était en outre possible de moduler la couleur des composés de formule (I) en faisant varier la nature et/ou la position des différents substituants R présents sur la molécule.

Les composés selon l'invention répondent donc à la formule générale (I) :

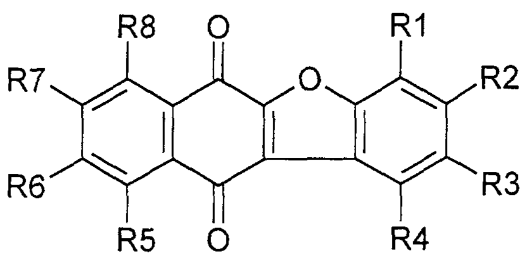

dans laquelle les radicaux R1 à R8 représentent, indépendamment les uns des autres :

- un atome d'hydrogène;
- un atome d'halogène (chlore, brome, iode, fluor);
- un radical hydroxy (-OH);
- un radical amino -NRR' avec R et R', indépendamment l'un de l'autre, représentent H ou un radical hydrocarboné, linéaire, ramifié ou cyclique, saturé ou insaturé, ayant 1 à 36 atomes de carbone;
- un radical nitro ($-NO_2$);
- un radical alkylamido -NH-CO-R" avec R" représentant un radical hydrocarboné, linéaire, ramifié ou cyclique, saturé ou insaturé, ayant 1 à 36 atomes de carbone;
- un radical uréido -NH-CO-NH-R"' avec R"' représentant H ou un radical hydrocarboné, linéaire, ramifié ou cyclique, saturé ou insaturé, ayant 1 à 36 atomes de carbone;
- un radical alkyluréthanne de formule -O-CO-NHR"" avec R"" représentant un radical hydrocarboné, linéaire, ramifié ou cyclique, saturé ou insaturé, ayant 1 à 36 atomes de carbone;
- un radical dialkylsiloxane de formule :

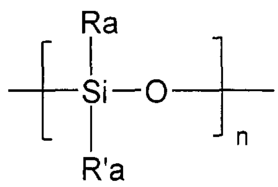

dans laquelle

- n est un entier compris entre 1 et 12;
- Ra et R'a, indépendamment l'un de l'autre, représentent un radical hydrocarboné, linéaire, ramifié ou cyclique, saturé ou insaturé, ayant 1 à 36 atomes de carbone, qui peut éventuellement comporter 1 à 12 hétéroatomes choisis parmi O, N, S et Si et/ou qui peut être substitué par un ou plusieurs substituants choisis dans la liste ci-dessous;

- un radical trialkylsilane de formule :

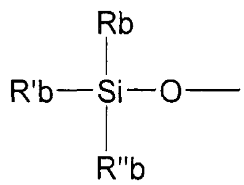

dans laquelle Rb, R'b et R"b, indépendamment l'un de l'autre, représentent un radical hydrocarboné, linéaire, ramifié ou cyclique, saturé ou insaturé, ayant 1 à 36 atomes de carbone, qui peut éventuellement comporter 1 à 12 hétéroatomes choisis parmi O, N, S et Si et/ou qui peut être substitué par un ou plusieurs substituants choisis dans la liste ci-dessous;
- un radical hydrocarboné, linéaire, ramifié ou cyclique, saturé ou insaturé, ayant 1 à 36 atomes de carbone, qui peut éventuellement comporter 1 à 12 hétéroatomes choisis parmi O, N, S et Si et/ou qui peut être substitué par un ou plusieurs substituants choisis dans la liste ci-dessous;
- les radicaux R1 et R2 ensemble et/ou R3 et R4 ensemble pouvant former, avec une chaîne hydrocarbonée, un cycle, saturé ou insaturé, comportant au total 5 ou 6 atomes de carbone, pouvant être lui-même interrompu par un ou plusieurs hétéroatomes choisis parmi O, N et S, et/ou substitué par un ou plusieurs substituants choisis dans la liste ci-dessous; ou
- les radicaux R2 et R3 ensemble pouvant former, avec une chaîne hydrocarbonée, un cycle, saturé ou insaturé, comportant au total 5 ou 6 atomes de carbone, pouvant être lui-même interrompu par un ou plusieurs hétéroatomes choisis parmi O, N et S, et/ou substitué par un ou plusieurs substituants choisis dans la liste ci-dessous.

Parmi les substituants susceptibles d'être portés par les composés ci-dessus, notamment par les radicaux hydrocarbonés des composés de formule (I) et/ou par les groupements alkyl des radicaux trialkylsilane ou dialkylsiloxane, on peut citer les halogènes, les radicaux hydroxy, amino, nitro, dialkylsiloxane ou trialkylsilane tels que ci-dessus définis; les radicaux hydrocarbonés, linéaires, ramifiés ou cycliques, saturés ou insaturés, ayant 1 à 36 atomes de carbone, éventuellement interrompus par 1 à 12 hétéroatomes choisis parmi O, N, S et Si et/ou eux-mêmes éventuellement substitués.
Parmi les radicaux hydrocarbonés en C1-36, éventuellement interrompus et/ou substitués, on peut citer les radicaux alkyle saturé ou insaturé en C1-36, les radicaux alcoxy (RO- avec R en C1-36), les radicaux acyloxy (R-CO-O- avec R en C1-36) et les radicaux acyle (R-CO- avec R en C1-36).
Notamment, les composés employés dans le cadre de l'invention peuvent correspondre à la formule (I) dans laquelle :

- les radicaux R1, R2, R3 et R4 représentent, indépendamment les uns des autres, un atome d'hydrogène ou un radical hydrocarboné, linéaire, ramifié ou cyclique, saturé ou insaturé, ayant 1 à 36 atomes de carbone, qui peut éventuellement comporter 1 à 8 hétéroatomes choisis parmi O ou N, et/ou qui peut être substitué;
- les radicaux R1 et R2 ensemble et/ou R3 et R4 ensemble forment, avec une chaîne hydrocarbonée, un cycle, saturé ou insaturé, comportant au total 6 atomes de carbone, pouvant être lui-même interrompu par un ou plusieurs

hétéroatomes et/ou substitué par un radical hydrocarboné, linéaire, ramifié ou cyclique, saturé ou insaturé, ayant 1 à 36 atomes de carbone, qui peut éventuellement comporter lui-même 1 à 8 hétéroatomes choisis parmi O ou N, et/ou qui peut être substitué par un radical hydrocarboné, linéaire, ramifié ou cyclique, saturé ou insaturé, ayant 1 à 36 atomes de carbone; et/ou

- les radicaux R2 et R3 ensemble forment, avec une chaîne hydrocarbonée, un cycle, saturé ou insaturé, comportant au total 6 atomes de carbone, pouvant être lui-même interrompu par un ou plusieurs hétéroatomes et/ou substitué par un radical hydrocarboné, linéaire, ramifié ou cyclique, saturé ou insaturé, ayant 1 à 36 atomes de carbone, qui peut éventuellement comporter lui-même 1 à 8 hétéroatomes choisis parmi O ou N, et/ou qui peut être substitué par un radical hydrocarboné, linéaire, ramifié ou cyclique, saturé ou insaturé, ayant 1 à 36 atomes de carbone.

En particulier, les composés de formule (I) peuvent être choisis parmi les composés répondant à l'une des cinq formules suivantes :

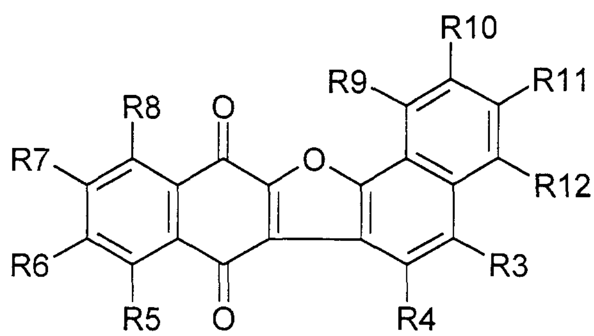

(II)

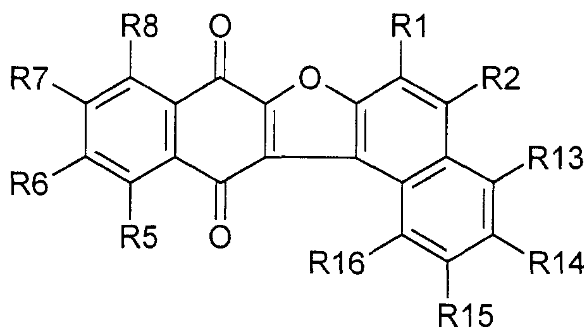

(III)

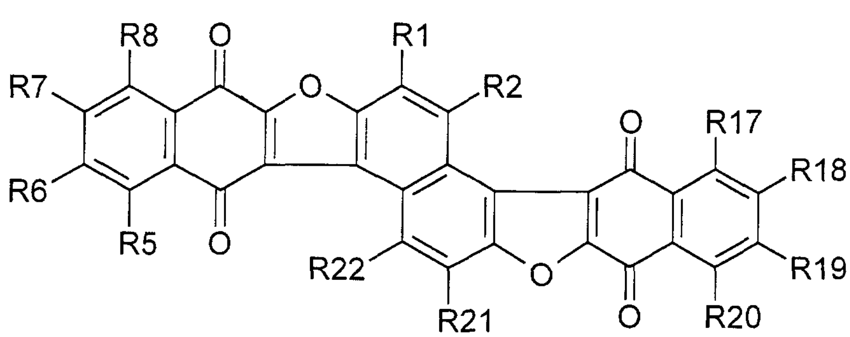

(IV)

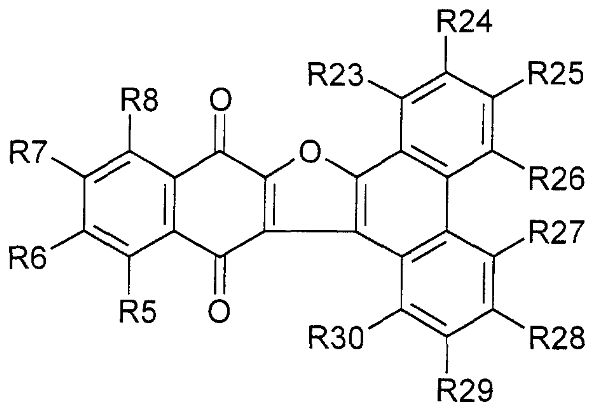

(V)

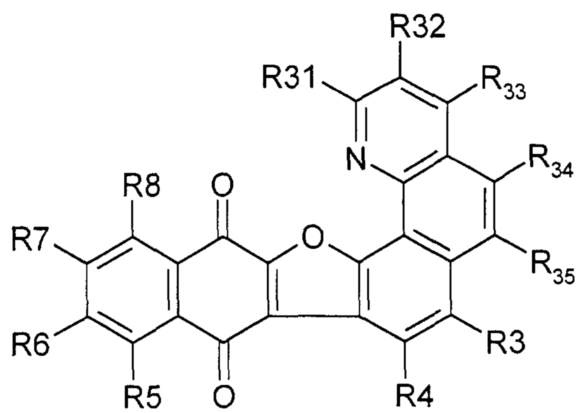

(VI)

dans lesquelles les radicaux R9 à R35 peuvent être identiques ou différents, et avoir les significations données pour les radicaux R1 à R8 ci-dessus, à savoir choisis parmi un atome d'hydrogène; un atome d'halogène; un radical hydroxy, amino, nitro, alkylamido, uréido, alkyluréthanne, dialkylsiloxane, trialkylsilane ou un radical hydrocarboné, linéaire, ramifié ou cyclique, saturé ou insaturé, ayant 1 à 36 atomes de carbone, qui peut éventuellement comporter un ou plusieurs hétéroatomes choisis parmi O, N, S et Si et/ou qui peut être substitué par un ou plusieurs substituants; étant entendu que deux radicaux adjacents peuvent former ensemble et avec une chaîne hydrocarbonée, un cycle, saturé ou insaturé, comportant au total 5 ou 6 atomes de carbone, pouvant être lui-même interrompu par un ou plusieurs hétéroatomes (O,N,S Si) et/ou substitué par un ou plusieurs substituants choisis dans la liste ci-dessus.

Les composés plus particulièrement préférés dans le cadre de l'invention sont choisis parmi les composés de formule (I), notamment de formules (II) à (VI) et plus préférentiellement parmi les composés de formule (II) et (III), dans lesquelles les différents radicaux sont choisis, indépendamment les uns des autres, parmi :

- un atome d'hydrogène;
- un atome d'halogène (chlore, brome, iode, fluor);
- un radical hydroxy (-OH);
- un radical alcoxy (RO-) avec R en C1-12, saturé ou insaturé, linéaire ou ramifié;
- un radical alkyle, linéaire ou ramifié, saturé ou insaturé en C1-12;
- un radical acyle (R-CO-) avec R en C1-12, saturé ou insaturé, linéaire ou ramifié;
- un radical amino -NRR' avec R et R', indépendamment l'un de l'autre, représentant H ou un radical hydrocarboné, linéaire ou ramifié, saturé ou insaturé, ayant 1 à 12 atomes de carbone;
- un radical nitro (-$NO_2$).

Encore plus préférentiellement, lesdits composés sont choisis parmi les composés de formule (I), notamment de formules (II) à (VI) et plus préférentiellement parmi les composés de formule (II) et (III), dans lesquelles les différents radicaux sont choisis, indépendamment les uns des autres, parmi :

- un atome d'hydrogène;

- un atome de chlore ou de brome;
- un radical hydroxy (-OH);
- un radical alcoxy (RO-) avec R en C1-6, saturé ou insaturé, linéaire ou ramifié, notamment méthoxy, éthoxy ou propoxy;
- un radical alkyle, linéaire ou ramifié, saturé ou insaturé en C1-6.

Parmi les composés particulièrement préférés, on peut citer :

- parmi les composés répondant à la formule (II) :

  - la dinaphto[1,2-b:2',3'-d]furan-7,12-dione
  - la 2-hydroxy-dinaphto[1,2-b:2',3'-d]furan-7,12-dione
  - la 3-hydroxy-dinaphto[1,2-b:2',3'-d]furan-7,12-dione
  - la 4-hydroxy-dinaphto[1,2-b:2',3'-d]furan-7,12-dione
  - la 5-méthoxy-dinaphto[1,2-b:2',3'-d]furan-7,12-dione
  - la 5-chloro-dinaphto[1,2-b:2',3'-d]furan-7,12-dione
  - la 5-éthoxy-dinaphto[1,2-b:2',3'-d]furan-7,12-dione
  - la 5-isopropyloxy-dinaphto[1,2-b:2',3'-d]furan-7,12-dione
  - la 5-hexyloxy-dinaphto[1,2-b:2',3'-d]furan-7,12-dione
  - la 5-[cholest-5-en-3β-ol]-dinaphto[1,2-b:2',3'-d]furan-7,12-dione

- parmi les composés répondant à la formule (III) :

  - la dinaphto[2,1-b:2',3'-d]furan-8,13-dione
  - la 2-méthoxy-dinaphto[2,1-b:2',3'-d]furan-8,13-dione
  - la 3-bromo-dinaphto[2,1-b:2',3'-d]furan-8,13-dione
  - la 3-hydroxy-dinaphto[2,1-b:2',3'-d]furan-8,13-dione
  - la 3-méthoxy-dinaphto[2,1-b:2',3'-d]furan-8,13-dione
  - la 5-hydroxy-dinaphto[2,1-b:2',3'-d]furan-8,13-dione

- parmi les composés répondant à la formule (IV):

  - la (dinaphto[2,1-b:2',3'-d]furan-8,13-dione)[3,4-b]naphto[2',3'-d]furan-5,14-dione

- parmi les composés répondant à la formule (V) :

  - la naphto[2,3-b]-phénanthro[9,10-d]furan-10,15-dione

- parmi les composés répondant à la formule (VI) :

  - la naphto[2,3-b]-5-azo-phenanthro[3',4'-d]furan-10,15-dione

Certains de ces composés sont nouveaux en tant que tels et forment donc un autre objet de l'invention. Ces composés répondent à l'une des formules (IIa) à (VIa) ci-dessous :

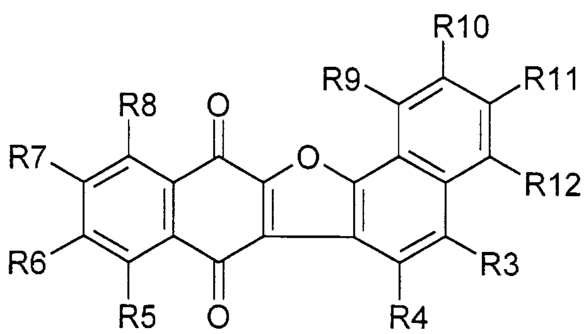

(IIa)

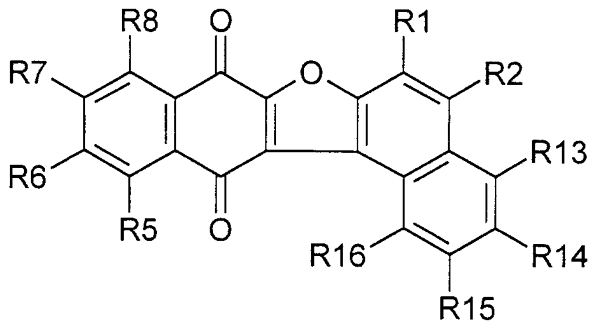

(IIIa)

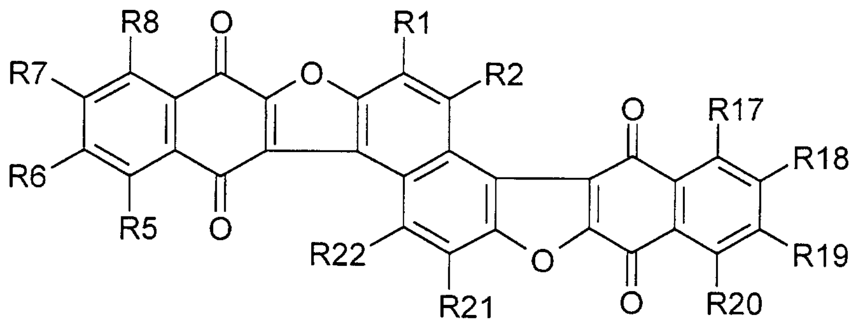

(IVa)

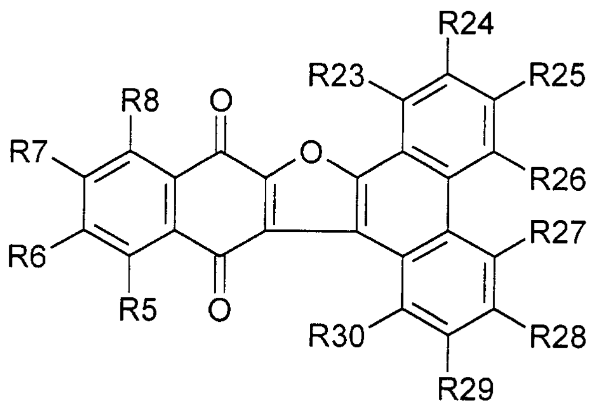

(Va)

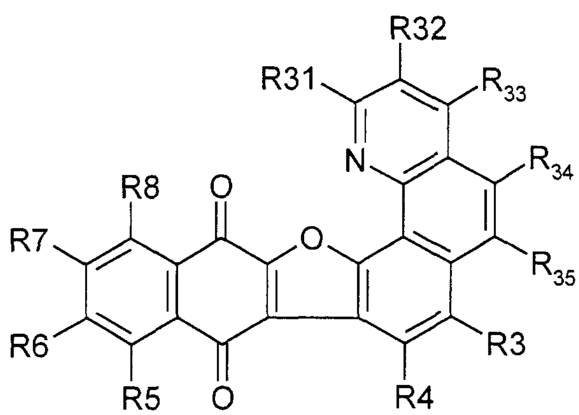

(VIa)

dans lesquelles les radicaux R9 à R35 peuvent être identiques ou différents, et avoir les significations données pour

les radicaux R1 à R8 ci-dessus, à savoir choisis parmi un atome d'hydrogène; un atome d'halogène; un radical hydroxy, amino, nitro, alkylamido, uréido, alkyluréthanne, dialkylsiloxane, trialkylsilane ou un radical hydrocarboné, linéaire, ramifié ou cyclique, saturé ou insaturé, ayant 1 à 36 atomes de carbone, qui peut éventuellement comporter un ou plusieurs hétéroatomes choisis parmi O, N, S et Si et/ou qui peut être substitué par un ou plusieurs substituants; étant entendu que deux radicaux adjacents peuvent former ensemble et avec une chaîne hydrocarbonée, un cycle, saturé ou insaturé, comportant au total 5 ou 6 atomes de carbone, pouvant être lui-même interrompu par un ou plusieurs hétéroatomes (O,N,S Si) et/ou substitué par un ou plusieurs substituants choisis dans la liste ci-dessus;
à l'exception des composés :

dans lesquelles les radicaux R9 à R35 peuvent être identiques ou différents, et sont choisi parmi les significations données pour les radicaux R1 à R8 dans l'une des revendications 1 à 2, à l'exception des composés :

- de formule (IIa) ou (IIIa) ou (Va) dans lesquelles tous les radicaux représentent H;
- de formule (IIa) dans laquelle R3 = OH et tous les autres radicaux représentent H;
- de formule (IIa) dans laquelle R3 = $OCH_3$ et tous les autres radicaux représentent H;
- de formule (IIa) dans laquelle R10 = OH et tous les autres radicaux représentent H;
- de formule (IIa) dans laquelle R11 = OH et tous les autres radicaux représentent H;
- de formule (IIIa) dans laquelle tous les radicaux représentent H;
- de formule (IIIa) dans laquelle R13 = $OCH_3$ et tous les autres radicaux représentent H;
- de formule (IIIa) dans laquelle R13 = OH et tous les autres radicaux représentent H;
- de formule (IIIa) dans laquelle $R_1$=OH et tous les autres radicaux représentent H;
- de formule (IIIa) dans laquelle R2=OH et tous les autres radicaux représentent H;
- de formule (IIIa) dans laquelle R1=$OCH_3$ et tous les autres radicaux représentent H;
- de formule (IIa) ou (IIIa) dans lesquelles $R_5$= $NO_2$ et tous les autres radicaux représentent H;
- de formule (IIa) ou (IIIa) dans lesquelles $R_8$ = $NO_2$ et tous les autres radicaux représentent H;
- de formule (IIa) ou (IIIa) dans lesquelles $R_5$ = $NH_2$ et tous les autres radicaux représentent H;
- de formule (IIa) ou (IIIa) dans lesquelles $R_8$ = $NH_2$ et tous les autres radicaux représentent H;
- de formule (IIa) ou (IIIa) dans lesquelles $R_5$ désigne benzamido et tous les autres radicaux représentent H;
- de formule (IIa) ou (IIIa) dans lesquelles $R_8$ désigne benzamido et tous les autres radicaux représentent H;
- de formule (IIIa) dans laquelle $R_8$= Br et tous les autres radicaux représentent H;
- de formule (IIIa) dans laquelle $R_8$= p-tolylsulfonamido et tous les autres radicaux représentent H;
- de formule (IIIa) dans laquelle $R_1$ =$OCH_3$ et tous les autres radicaux représentent H;
- de formule (IIIa) dans laquelle $R_2$ = O(CH2)2 - $N(CH_3)_2H.Cl$ et tous les autres radicaux représentent H;
- de formule (IIIa) dans laquelle $R_2$ = $O(CH_2)_3$ - $N(CH_3)_2H.Cl$ et tous les autres radicaux représentent H;
- de formules (IIIa) dans lesquelles $R_1$ est choisi parmi -CONH-(2'- pyridyl),-CONH-(2'- pyrimidinyl), -CONH-(2'-thiazolyl), -CONH-(3'(H-1,2,4-triazolyl) et-CONH-phényl et tous les autres radicaux représentent H;
- de formules (IIIa) dans lesquelles $R_1$ est choisi parmi -CONH-(2'- méthylphényl), -CONH-(4'- cyanophényl), -CONH-(2'(3'-méthoxypyridyl) et -CONH-(4'-méthoxyphényl), R14 et R15 désignent ensemble -CH=CH-CH=CH- et tous les autres radicaux représentent H;
- de formules (IIIa) dans lesquelles $R_1$ a la formule suivante :

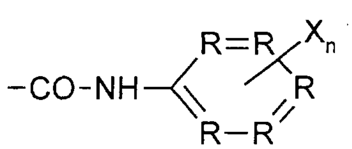

dans laquelle R est choisi parmi N et CH, de 1 à 3 radicaux R désignent N X est choisi parmi H, $CH_3$, $C_2H_5$, $NO_2$, $OCH_3$, CN, $SO_2NH_2$, $CO_2CH_3$, $CO_2C_2H_5$, $SO_2NHC_6H_5$, Cl, F, Br et I,
n est un entier positif de 1 à 4.

Ces composés sont notamment :

- la 5-chloro-dinaphto[1,2-b:2',3'-d]furan-7,12-dione,
- la 5-isopropyloxy-dinaphto[1,2-b:2',3'-d]furan-7,12-dione,
- la 2-méthoxy-dinaphto[2,1-b:2',3'-d]furan-8,13-dione,
- la 3-bromo-dinaphto[2,1-b:2',3'-d]furan-8,13-dione,

- la 5-hexyloxy-dinaphto[1,2-b:2',3'-d]furan-7,12-dione,
- la 5-[cholest-5-en-3β-ol]-dinaphto[1,2-b:2',3'-d]furan-7,12-dione,
- la (dinaphto[2,1-b:2',3'-d]furan-8,13-dione)[3,4-b]naphto[2',3'-d]furan-5,14-dione,
- la 3-méthoxy-dinaphto[2,1-b:2',3'-d]furan-8,13-dione,
- la naphto[2,3-b]-5-azo-phenanthro[3',4'-d]furan-10,15-dione.

Les composés de formule (I) peuvent être préparés par l'homme du métier sur la base de ses connaissances générales.
Notamment, ces composés peuvent être préparés à partir de 1-naphtols de la manière décrite dans la publication de Shand et al. Tetrahedron, 1963, vol.19, pp 1919-1937, qui décrit la réaction de 2,3-dicholor-1,4-naphtoquinone avec le 4-méthoxy-1-naphtol, au reflux dans la pyridine.
Les composés de formule (I) peuvent être utilisés, notamment comme agent colorant, dans une composition notamment cosmétique qui peut se présenter sous la forme d'un produit à appliquer sur les muqueuses, les semi-muqueuses et/ou les tissus kératiniques tels que la peau et les phanères (ongles, cils, sourcils, poils et cheveux).
Les composés de formule (I) peuvent être incorporés dans la composition notamment cosmétique, en une quantité aisément déterminable par l'homme du métier sur la base de ses connaissances générales, et qui peut notamment être comprise entre 0,01 à 50% en poids par rapport au poids total de la composition, de préférence en une quantité de 0,1 à 20% en poids, notamment de 0,5 à 10% en poids.
On a en effet constaté que les composés selon l'invention présentaient de bonnes propriétés de coloration, notamment une force colorante, une stabilité photochimique, ainsi qu'un faible dégorgement dans l'eau, adéquats pour une telle utilisation. Par ailleurs, ils possèdent également une bonne couvrance et une bonne stabilité thermique.
Par force colorante, on entend l'aptitude d'un composé à teinter une masse blanche en dispersion dans un milieu huileux. La force colorante est généralement mesurée au moyen d'un colorimètre et s'exprime par l'écart de couleur du composé pur et du composé en dispersion. Les composés selon l'invention présentent notamment une force colorante comparable, voire supérieure, à celle des pigments de la même teinte usuellement employés en cosmétique.
Par dégorgement, on entend la propriété qu'a un composé à se solubiliser dans le milieu dans lequel il est dispersé, de sorte qu'il le colore. On peut le quantifier par mesure de la densité optique du surnageant saturé en colorant.
Les composés selon l'invention présentent notamment un dégorgement comparable, voire supérieur, à celui des pigments de la même teinte usuellement employés en cosmétique.
Par couvrance, on entend l'aptitude qu'a un composé dispersé dans un milieu huileux, à masquer une plaque de contraste à damiers blancs et noirs. Les composés selon l'invention présentent, pour certains, une bonne couvrance.
Par ailleurs, les composés selon l'invention présentent notamment de préférence une bonne stabilité thermique. Ainsi ils sont généralement stables après 24 heures à 90°C et après 2 mois à 60°C.
Ces composés présentent également une bonne stabilité photochimique. On entend par stabilité photochimique l'aptitude qu'à un composé à ne pas se colorer sous irradiation UV. On quantifie la photostabilité par détermination de la variation colorimétrique entre le composé pur compacté avant et après irradiation UV. Les composés selon l'invention présentent notamment une stabilité photochimique comparable, voire supérieure, à celle des pigments de référence dans la teinte, usuellement employés en cosmétique.
Les méthodes de mesure sont données avant les exemples.
Ladite composition contient donc un milieu cosmétiquement acceptable, c'est-à-dire un milieu compatible avec toutes les matières kératiniques telles que la peau, les ongles, les cheveux, les cils et sourcils, les muqueuses et les semi-muqueuses, et toute autre zone cutanée du corps et du visage.
Ledit milieu peut comprendre ou se présenter sous la forme de, notamment, une suspension, une dispersion, une solution en milieu solvant ou hydroalcoolique, éventuellement épaissie voire gélifiée; une émulsion huile-dans-eau, eau-dans-huile, ou multiple; un gel ou une mousse; un gel émulsionné; une dispersion de vésicules notamment lipidiques; une lotion biphase ou multiphase; un spray; une poudre libre, compacte ou coulée; une pâte anhydre. L'homme du métier pourra choisir la forme galénique appropriée, ainsi que sa méthode de préparation, sur base de ses connaissances générales, en tenant compte d'une part de la nature des constituants utilisés, notamment de leur solubilité dans le support, et d'autre part de l'application envisagée pour la composition.
Lorsque la composition se présente sous forme aqueuse, notamment sous forme de dispersion, d'émulsion ou de solution aqueuse, elle peut comprendre une phase aqueuse, qui peut comprendre de l'eau, une eau florale et/ou une eau minérale.
Ladite phase aqueuse peut comprendre en outre des alcools tels que des monoalcools en $C_1$-$C_6$ et/ou des polyols tels que le glycérol, le butylèneglycol, l'isoprène glycol, le propylèneglycol, le polyéthylèneglycol.
Lorsque la composition selon l'invention se présente sous la forme d'une émulsion, elle peut éventuellement comprendre en outre un tensioactif, de préférence en une quantité de 0,01 à 30% en poids par rapport au poids total de la composition. La composition selon l'invention peut également comprendre au moins un coémulsionnant qui peut être choisi parmi le monostéarate de sorbitan oxyéthyléné, des alcools gras tels que l'alcool stéarylique ou l'alcool cétylique,

ou des esters d'acides gras et de polyols tels que le stéarate de glycéryle.
La composition selon l'invention peut encore comprendre un ou plusieurs agents épaississants dans des concentrations préférentielles allant de 0 à 6 % en poids, par rapport au poids total de la composition, choisis parmi:

- les biopolymères polysaccharidiques comme la gomme de xanthane, la gomme de caroube, la gomme de guar, les alginates, les celluloses modifiées, les dérivés de l'amidon, les dérivés d'éthers de cellulose possédant des groupes ammonium quaternaires, les polysaccharides cationiques;
- les polymères synthétiques comme les acides polyacryliques, la polyvinylpyrrolidone, l'alcool polyvinylique, les polymères à base de polyacrylamide
- le silicate de magnésium et d'aluminium.

Selon l'application envisagée, la composition peut comprendre en outre un polymère filmogène. Ceci est notamment le cas lorsque l'on souhaite préparer une composition de type vernis à ongles, mascara, eye-liner ou composition capillaire de type laque. Les polymères peuvent être dissous ou dispersés dans le milieu cosmétiquement acceptable. En particulier, le polymère peut être présent sous forme de solution dans un solvant organique ou sous forme de dispersion aqueuse de particules de polymère filmogène. Ledit polymère peut être choisi parmi la nitrocellulose, l'acétobutyrate de cellulose, les butyralpolyvinyliques, les résines alkydes, les polyesters, les acryliques, les vinyliques, et/ou les polyuréthannes.
La composition peut également comprendre au moins un plastifiant, qui peut être présent à une teneur allant de 1 % à 40 % en poids par rapport au poids total de la composition.
La composition selon l'invention peut également comprendre une phase grasse, notamment constituée de corps gras liquides à 25°C, tels que des huiles d'origine animale, végétale, minérale ou synthétique; de corps gras solides à 25°C tels que des cires d'origine animale, végétale, minérale ou synthétique; de corps gras pâteux; de gommes; de leurs mélanges.
Les compositions selon l'invention peuvent ainsi comprendre des huiles volatiles, qui s'évaporeront au contact de la peau, mais dont la présence, dans la composition cosmétique, est utile car elles facilitent l'étalement de la composition lors de l'application sur la peau. De tels agents d'étalement appelés ici "huiles volatiles", sont généralement des huiles ayant, à 25°C, une tension de vapeur saturante au moins égale à 0,5 millibar (soit 50 Pa).
On peut ainsi citer les huiles siliconées volatiles, telles que :

- les silicones volatiles cycliques ayant de 3 à 8 atomes de silicium et de préférence de 4 à 6.
- les cyclocopolymères du type diméthylsiloxane/méthylalkylsiloxane,
- les silicones volatiles linéaires ayant de 2 à 9 atomes de silicium.

On peut également citer les huiles volatiles hydrocarbonées, telles que les isoparaffines et notamment l'isododécane et des huiles fluorées.
On peut également utiliser des huiles non volatiles, parmi lesquelles on peut citer:

- les polyalkyl($C_1$-$C_{20}$) siloxanes et notamment ceux à groupements terminaux triméthylsilyle, parmi lesquels on peut citer les polydiméthylsiloxanes linéaires et les alkylméthylpolysiloxanes tels que la cétyldiméthicone (nom CTFA),
- les silicones modifiées par des groupements aliphatiques et/ou aromatiques, éventuellement fluorés, ou par des groupements fonctionnels tels que des groupements hydroxyles, thiols et/ou amines.
- les huiles de silicone phénylées, notamment celles de formule :

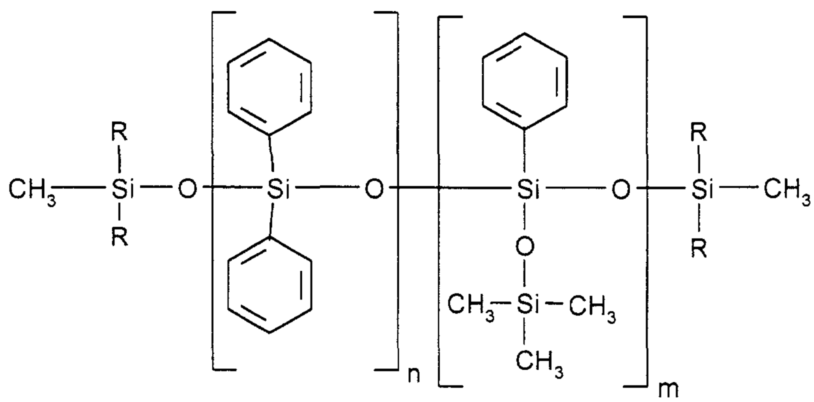

dans laquelle R est un radical alkyle en C1-C30, un radical aryle ou un radical aralkyle, n est un nombre entier compris entre 0 et 100, et m est un nombre entier compris entre 0 et 100, sous réserve que la somme est comprise entre 1 et 100.

- les huiles d'origine animale, végétale ou minérale, et notamment les huiles animales ou végétales formées par des esters d'acide gras et de polyols, en particulier les triglycérides liquides, par exemple les huiles de tournesol, de maïs, de soja, de courge, de pépins de raisin, de sésame, de noisette, d'abricot, d'amandes ou d'avocat; les huiles de poisson, le tricaprocaprylate de glycérol, ou les huiles végétales ou animales de formule $R1COOR_2$ dans laquelle $R_1$ représente le reste d'un acide gras supérieur comportant de 7 à 19 atomes de carbone et $R_2$ représente une chaîne hydrocarbonée ramifiée contenant de 3 à 20 atomes de carbone, par exemple, l'huile de Purcellin; l'huile de paraffine, de vaseline, le perhydrosqualène, l'huile de germes de blé, de calophyllum, de sésame, de macadamia, de pépins de raisin, de colza, de coprah, d'arachide, de palme, de ricin, de jojoba, d'olive ou de germes de céréales; des esters d'acides gras; des alcools; des acétylglycérides; des octanoates, décanoates ou ricinoléates d'alcools ou de polyalcools; des triglycérides d'acides gras; des glycérides;
- les huiles fluorées et perfluorées.

La composition selon l'invention peut comprendre en outre d'autres corps gras, qui peuvent être choisis par l'homme du métier sur base de ses connaissances générales, de manière à conférer à la composition finale les propriétés souhaitées, par exemple en consistance et/ou en texture. Ces corps gras additionnels peuvent être des cires, des gommes et/ou des corps gras pâteux d'origine animale, végétale, minérale ou synthétique, ainsi que leurs mélanges. On peut notamment citer les gommes de silicones; les cires d'origine animale, végétale, minérale ou synthétique telles que les cires microcristallines, la paraffine, le pétrolatum, la vaseline, l'ozokérite, la cire de montan; la cire d'abeilles, la lanoline et ses dérivés; les cires de Candellila, d'Ouricury, de Carnauba, du Japon, le beurre de cacao, les cires de fibres de lièges ou de canne à sucre; les huiles hydrogénées concrètes à 25°C, les ozokérites, les esters gras et les glycérides concrets à 25°C; les cires de polyéthylène et les cires obtenues par synthèse de Fischer-Tropsch; des huiles hydrogénées concrètes à 25°C; des lanolines; des esters gras concrets à 25°C; les cires de silicone; les cires fluorées.

La composition peut comprendre en outre une phase particulaire, qui peut comprendre des pigments et/ou des nacres et/ou des charges habituellement utilisés dans les compositions cosmétiques. Les pigments peuvent être présents dans la composition à raison de 0 à 15% en poids de la composition finale, de préférence à raison de 8 à 10% en poids, et peuvent être blancs ou colorés, minéraux ou organiques, de taille usuelle ou nanométrique. On peut citer les dioxydes de titane, de zirconium ou de cérium, ainsi que les oxydes de zinc, de fer ou de chrome, le bleu ferrique, l'hydrate de chrome, les outremers (polysulfures d'aluminosilicates), le pyrophosphate de manganèse et certaines poudres métalliques telles que celles d'argent ou d'aluminium, le noir de carbone. On peut encore citer les laques couramment employées pour conférer aux lèvres et à la peau un effet de maquillage, qui sont des sels de calcium, de baryum, d'aluminium ou de zirconium, de colorants acides.

Les nacres peuvent être présentes dans la composition à raison de 0 à 20% en poids, de préférence 8 à 15% en poids, et peuvent être choisies parmi la nacre naturelle, le mica recouvert d'oxyde de titane, d'oxyde de fer, de pigment naturel ou d'oxychlorure de bismuth ainsi que le mica titane coloré.

Les charges, qui peuvent être présentes à raison de 0 à 30% en poids, de préférence 5 à 15%, dans la composition, peuvent être minérales ou de synthèse, lamellaires ou non lamellaires. On peut citer le talc, le mica, la silice, le kaolin, les poudres de Nylon et de polyéthylène, le Téflon, l'amidon, le nitrure de bore, les microsphères de polymère telles que l'Expancel (Nobel Industrie), le polytrap (Dow Corning) et les microbilles de résine de silicone (Tospearls de Toshiba, par exemple), le carbonate de calcium précipité, le carbonate ou l'hydrocarbonate de magnésium, des savons

métalliques dérivés d'acides organiques carboxyliques ayant de 8 à 22 atomes de carbone.
La composition peut en outre comprendre un colorant hydrosoluble ou liposoluble, notamment un colorant organique naturel tel que le carmin de cochenille, et/ou un colorant de synthèse tel que les colorants halogéno-acides, azoïques, anthraquinoniques. On peut également citer des colorants minéraux tels que le sulfate de cuivre.

La composition peut comprendre en outre tout additif usuellement utilisé dans le domaine cosmétique, tel que des antioxydants, des parfums, des huiles essentielles, des conservateurs, des actifs cosmétiques lipophiles ou hydrophiles, des hydratants, des vitamines, des acides gras essentiels, des sphingolipides, des agents autobronzants tels que la DHA, des filtres solaires, des agents antimousses, des agents séquestrants, des antioxydants. Bien entendu l'homme du métier veillera à choisir les éventuels composés complémentaires et leur quantité, de manière telle que les propriétés avantageuses de la composition selon l'invention ne soient pas, ou substantiellement pas, altérées par l'adjonction envisagée.

Les compositions cosmétiques selon l'invention peuvent se présenter

- sous la forme d'un produit de maquillage de la peau du visage, du corps ou des lèvres ou des fibres kératiniques (ongles, cils, sourcils, cheveux), tel qu'un fond de teint, une crème teintée, un fard à joues ou à paupières, une poudre libre ou compacte, un stick anti-cernes, un stick camouflant, un eye-liner, un mascara, un rouge à lèvres, un vernis-à-ongles, une composition de maquillage des cheveux;
- sous la forme d'un produit de soin de la peau du visage ou du corps y compris le cuir chevelu, tel qu'une composition de soin (de jour, de nuit, anti-rides, hydratante, etc.) pour le visage; une composition matifiante pour le visage;
- sous la forme d'une composition de protection solaire ou de bronzage artificiel (autobronzant);
- sous la forme d'une composition capillaire, et notamment une crème ou un gel coiffant, une composition de teinture, d'oxydation ou directe, éventuellement sous forme de shampooing colorant;

L'invention est illustrée plus en détail dans les exemples suivants.

**<u>Méthodes de mesure</u>**

Le colorimètre employé est un colorimètre CR-300 de Minolta.

<u>1/ force colorante : aptitude à teinter une masse blanche ($TiO_2$) en dispersion dans un milieu huileux</u>

- mesure sur le composé pur : on compacte le composé pur à 100 bars, dans une coupelle FAP.
- mesure du composé en dispersion : on prépare un mélange comprenant :
  - vaseline 10 g
  - oxyde de titane (non traité) 4 g
  - composé à tester 2 g

On homogénéise le mélange avec une spatule. On broye à la tricylindre (écartement sur 1) en faisant 3 passages.
Pour la mesure, le mélange est écrasé sous lame de verre, d'une épaisseur d'une lame de microscope.

On détermine les valeurs de (L, a, b) au moyen du colorimètre, pour le composé pur compacté et pour le mélange (vaseline + oxyde de titane + composé).
Dans le système (L, a, b), L représente la luminance, a représente l'axe rouge-vert (-a = vert, +a = rouge) et b représente l'axe jaune-bleu (-b =bleu, +b =jaune).
On détermine également la valeur C qui rend compte de la saturation du composé.

<u>2/ couvrance : aptitude à masquer une plaque de contraste à damiers</u>

On prépare une émulsion huile-dans-eau comprenant :

- huile de parléam 22 g
- acide stéarique 1,5 g
- Tween 60 (ICI) 0,9 g
- Sipol C16 (Henkel) 0,5 g
- Simulsol 165 (Seppic) 2,1 g

- Triéthanolamine 0,75 g
- composé à tester 10 g
- propylène glycol 3 g
- cyclopentadiméthylsiloxane 3 g
- Carbopol 981 0,15 g
- Rhodicare (Rhodia) 0,2 g
- eau qsp 100 g

On effectue trois passages à la tricylindre.
L'émulsion est appliquée sur les feuilles de contraste (damiers à cases blanches et noires alternées); pour cela on utilise un étaleur en appliquant une force homogène (90 µm) tout le long de la carte.
On détermine les valeurs de (L, a, b) au moyen du colorimètre, sur fond blanc et sur fond noir.
$\Delta E^*$ est calculé à partir des variations $\Delta L$, $\Delta a$ et $\Delta b$ selon la formule suivante :

$$\Delta E^* = \sqrt{(\Delta L)^2 + (\Delta a)^2 + (\Delta b)^2}$$

La couvrance $\Delta E^*$ correspond à la différence colorimétrique entre la mesure sur fond blanc et celle sur fond noir. Lorsque $\Delta E^* < 5$, le produit est considéré comme très couvrant; lorsque $\Delta E^* > 20$ le produit est considéré comme peu couvrant.

3/ dégorgement : aptitude à se solubiliser dans un milieu de dispersion

On prépare une dispersion comprenant 1% en poids de composé à tester dans une huile (triglycérides de l'acide caprylique/caprique).
On prépare également une dispersion comprenant 1% en poids de composé à tester dans un mélange eau + 1% en poids d'agent mouillant (Polysorbate 20).
On agite pendant 10 minutes au barreau magnétique. On pèse 10 g de dispersion que l'on dispose dans des tubes à centrifugation. On centrifuge pendant 30 minutes à 3000 tours/min.
On prélève le liquide surnageant et l'on détermine la densité optique par mesure de l'absorbance au spectrophotomètre de 400 à 800 nm, à une vitesse de 240 nm/min.
Lorsque la densité optique est supérieure à 1,5, on considère que le composé dégorge.

4/ stabilité photochimique

La stabilité aux U.V. s'effectue en laissant le composé à tester pur compacté (100 bars, coupelle FAP), pendant une heure au Suntest.
On mesure par colorimétrie la différence de couleur $\Delta E$ avant et après passage au Suntest.
Lorsque le $\Delta E$ est >6, le composé est considéré comme instable aux UV.

5/ Diffraction RX :

Le spectre DX caractérise la forme polymorphique des composés.

- Diffractomètre Siemens D5005 ; détecteur à scintillations
- Anticathode cuivre, voltage 40 KV, intensité 40 mA
- Montage $\theta$ - $\theta$
- Domaine de mesures : 5° à 30 ° (échantillon fixe)
- Incrémentation entre chaque mesure : 0,04°
- Temps de mesure par pas : 4s
- Température (20 ± 1)°C
- Fentes fixes : 1,6 mm
- Filtre K$\beta$ (Ni)
- Pas de référence interne
- Procédure de zéro avec les fentes Siemens
- Données expérimentales traitées avec le logiciel EVA (v 5.0)

**Exemple 1 : préparation de la 5-chloro-dinaphto[1,2-b:2',3'-d]furan-7,12-dione**

Formule :

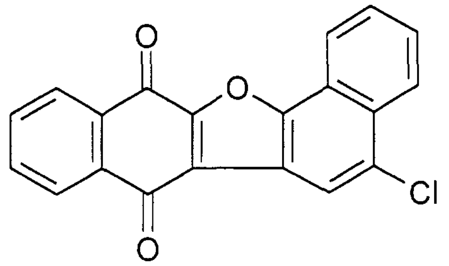

Dans un tricol de 500 ml, on solubilise à température ambiante 17,86 g (0,1 mol) de 4-chloro-naphtalèn-1-ol et 22,71 g (0,1 mol) de 2,3-dichloro-[1,4]naphtoquinone dans 300 ml de pyridine. On porte le mélange sous agitation au reflux du solvant pendant 3 heures. On refroidit le mélange réactionnel à 10°C pendant 30 minutes et on filtre le solide sur verre fritté. On lave les cristaux à l'eau, l'éthanol et l'éther isopropylique; on sèche sous vide.
On obtient 30,86 g (rendement : 93%) de 5-chloro-dinaphto[1,2b:2',3'-d]furan-7,12-dione sous forme de cristaux jaune vif.

- point de fusion : 276,4°C (DSC)
- HPCCM : monospot
- RMN $^1H$ ($CDCl_3$) : conforme
- Analyse élémentaire :

| | C | H | O | Cl |
|---|---|---|---|---|
| théorique | 72,20 | 2,73 | 14,43 | 10,65 |
| expérimental | 72,40 | 2,67 | 14,71 | 10,74 |

- spectre UV-Visible : $CHCl_3$: $\lambda_{max}$ nm (ε) : 438,5(74400); 341,5(39600); 284,0 (193500); 265,0(861300); 222,5 (221900); 208,0(209300)
- spectre DX

| ANGLE 2 (°) | d (Angstrom) | Intensité (count) | Intensité (%) |
|---|---|---|---|
| 8,858 | 9,97422 | 1212 | 91,4 |
| 10,545 | 8,38269 | 974 | 73,5 |
| 12,759 | 6,93251 | 1326 | 100 |
| 14,369 | 6,15885 | 366 | 27,6 |
| 15,21 | 5,8203 | 194 | 14,6 |
| 17,439 | 5,08119 | 159 | 12 |
| 17,822 | 4,97271 | 143 | 10,8 |
| 19,769 | 4,48716 | 252 | 19 |
| 21,228 | 4,18188 | 173 | 13 |
| 21,813 | 4,07116 | 126 | 9,5 |
| 22,346 | 3,97513 | 295 | 22,2 |
| 24,67 | 3,60578 | 247 | 18,6 |
| 24,927 | 3,56909 | 209 | 15,8 |

(suite)

| ANGLE 2 (°) | d (Angstrom) | Intensité (count) | Intensité (%) |
|---|---|---|---|
| 25,992 | 3,42522 | 858 | 64,7 |
| 26,525 | 3,3576 | 393 | 29,6 |
| 27,151 | 3,28166 | 692 | 52,2 |
| 28,096 | 3,17331 | 111 | 8,4 |
| 29,412 | 3,03425 | 100 | 7,5 |

**Exemple 2 : préparation de la 5-isopropyloxy-dinaphto[1,2-b:2',3'-d]furan-7,12-dione**

Formule :

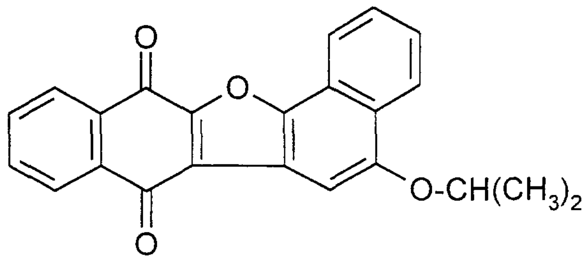

Dans un tricol de 1 litre, on solubilise 33,1 g (0,146 mol) de 2,3-dichloro-[1,4] naphtoquinone et 29,5 g (0,146 mol) de 4-isopropoxy-naphthalèn-1-ol dans 425 ml de pyridine. La solution est portée à 80°C sous agitation pendant 16 heures. On refroidit à 5°C et filtre le solide sur verre fritté. On lave les cristaux à l'eau puis au méthanol et à l'éthanol pour obtenir après séchage 34,4 g (rendement :66%) de 5-isopropyloxy-dinaphto[1,2-b:2',3'-d]furan-7,12-dione sous forme de cristaux rouge vif.

- point de fusion : 252,9°C (DSC)
- HPCCM : monospot
- RMN $^1H$ ($CDCl_3$): conforme
- Analyse élémentaire :

| | C | H | O |
|---|---|---|---|
| Théorique | 77,52 | 4,53 | 17,96 |
| expérimental | 77,52 | 4,47 | 17,90 |

- spectre UV-Visible : $CHCl_3$: $\lambda_{max}$ nm (ε) : 486,0(66200), 383,0(16400), 263,5 (510800), 235,0(254200), 203,0 (215700).

**Exemple 3 : préparation de la 2-méthoxy-dinaphto[2,1-b:2',3'-d]furan-8,13-dione**

Formule :

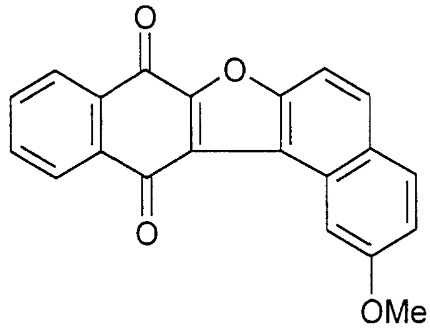

Dans un tricol de 1 litre, sous agitation, on dissout 34,05 g (0,15 mol) de 2,3-dichloro-[1,4]naphtoquinone et 26,10 g (0,15 mol) de 7-méthoxy-naphthalèn-2-ol dans 600 ml de pyridine et on chauffe 15 heures à 80°C, puis 2 heures au reflux du solvant. On refroidit à 5°C, on filtre le solide qui est lavé à l'eau, puis au méthanol et à l'éther diisopropylique. On sèche sous vide.
On obtient 40,27 g (rendement : 82%) de 2-méthoxy-dinaphto[2,1b:2',3'-d]furan-8,13-dione sous forme de cristaux rouge orangé.

- point de fusion : 264,2°C (DSC)
- HPCCM : monospot
- RMN $^1H$ ($CDCl_3$) : conforme
- Analyse élémentaire :

| | C | H | O |
|---|---|---|---|
| Théorique | 76,83 | 3,68 | 19,49 |
| expérimental | 76,35 | 3,64 | 19,41 |

- spectre UV-Visible : $CHCl_3$: $\lambda_{max}$ nm (ε) : 467,0(59700), 322,0(249900), 285,5 (208000), 246,5(477600), 222, 0 (195500), 205,5(199800).
- Spectre DX

| **ANGLE 2 (°)** | **d (Angstrom)** | **Intensité (count)** | **Intensité (%)** |
|---|---|---|---|
| 7,124 | 12,39813 | 1065 | 69,8 |
| 10,087 | 8,76195 | 1055 | 69,1 |
| 10,694 | 8,26606 | 1526 | 100 |
| 12,37 | 7,14936 | 890 | 58,3 |
| 13,022 | 6,79302 | 273 | 17,9 |
| 14,341 | 6,17099 | 453 | 29,7 |
| 14,784 | 5,98695 | 572 | 37,5 |
| 16,572 | 5,34479 | 214 | 14 |
| 16,696 | 5,30553 | 221 | 14,5 |
| 17,8 | 4,97885 | 198 | 13 |
| 18,612 | 4,76353 | 166 | 10,9 |
| 19,125 | 4,63688 | 304 | 19,9 |
| 19,56 | 4,53465 | 251 | 16,4 |
| 20,39 | 4,35189 | 223 | 14,6 |
| 20,653 | 4,2971 | 309 | 20,2 |

(suite)

| ANGLE 2 (°) | d (Angstrom) | Intensité (count) | Intensité (%) |
|---|---|---|---|
| 22,245 | 3,99296 | 337 | 22,1 |
| 23,524 | 3,77866 | 132 | 8,7 |
| 24,553 | 3,62271 | 168 | 11 |
| 25,317 | 3,51502 | 960 | 56,4 |
| 25,901 | 3,4371 | 1255 | 82,2 |
| 26,2 | 3,39852 | 819 | 53,7 |
| 27,568 | 3,23294 | 393 | 25,8 |
| 27,678 | 3,22028 | 420 | 27,5 |
| 29,622 | 3,01328 | 178 | 11,7 |

**Exemple 4 : préparation de la 3-bromo-dinaphto[2,1-b:2',3'-d]furan-8,13-dione**

Formule:

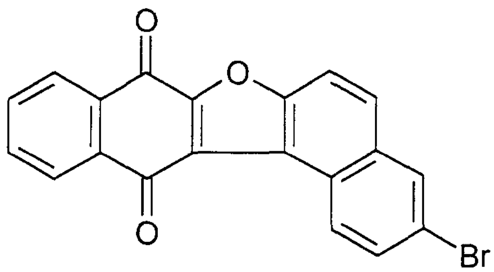

Dans un tricol de 1 litre, sous agitation, on dissout 29,23 g (0,13 mol) de 2,3-dichloro-[1,4]naphtoquinone et 28,71 g (0,13 mol) de 6-bromo-naphthalèn-2-ol dans 515 ml de pyridine. On chauffe le mélange à 80°C pendant 18 heures, puis on le refroidit à 5°C et on filtre le solide formé. On lave les cristaux à l'eau, puis au méthanol et à l'éther diisopropylique. On sèche sous vide.
On obtient 40,35 g (rendement :83%) de 3-bromodinaphto[2,1-b:2',3'-d]furan-8,13-dione sous forme de cristaux jaune orangé.

- point de fusion : 329,5°C (DSC)
- HPCCM : monospot
- RMN $^1H$ ($CDCl_3$) : conforme
- Analyse élémentaire :

| | C | H | O | Br |
|---|---|---|---|---|
| Théorique | 63,71 | 2,35 | 12,85 | 20,93 |
| Expérimental | 63,47 | 2,34 | 12,86 | 20,91 |

- spectre UV-Visible : $CHCl_3$: $\lambda_{max}$ nm (ε) : 443,0(79500), 295,5(295600), 284,5 (282000), 254,0(434700), 242,0 (384900), 229,0(234500), 208,5(211100)

**Exemple 5 : préparation de la 3-méthoxy-dinaphto[2,1-b:2',3'-d]furan-8,13-dione**

Formule :

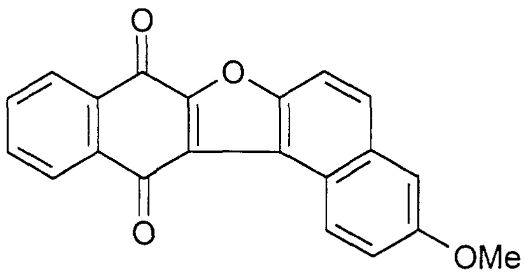

Dans un tricol de 1 litre, on solubilise 30,19 g (0,133 mol) de 2,3-dichloro-[1,4] naphtoquinone et 23,14 g (0,133 mol) de 6-méthoxy-naphthalèn-1-ol dans 400 ml de pyridine. La solution est portée à 85°C sous agitation pendant 18 heures. On refroidit à 5°C et on filtre le solide sur verre fritté. On lave les cristaux à l'eau puis au méthanol et à l'éther diisopropylique, et on sèche.

On obtient 24,8 g (rendement : 57%) de 3-méthoxy-dinaphto[2,1-b:2',3'-d]furan-8,13-dione sous forme de cristaux orange.

- point de fusion : 292,1°C (DSC)
- HPCCM : monospot
- RMN $^1H$ ($CDCl_3$) : conforme
- Analyse élémentaire :

| | C | H | O |
|---|---|---|---|
| théorique | 76,83 | 3,68 | 19,49 |
| expérimental | 76,37 | 3,60 | 19,34 |

- spectre UV-Visible : $CHCl_3$: $\lambda_{max}$ nm (ε) : 467,0(104300), 319,0(110500), 296,0 (430700), 284,5(288400), 257,0 (596300), 230,5(280400).
- Spectre DX

| ANGLE 2 (°) | d (Angstrom) | Intensité (count) | Intensité (%) |
|---|---|---|---|
| 7,02 | 12,5816 | 1800 | 73,2 |
| 10,555 | 8,3741 | 621 | 25,2 |
| 11,834 | 7,47227 | 2460 | 100 |
| 12,213 | 7,24081 | 976 | 39,7 |
| 12,926 | 6,89616 | 1812 | 73,7 |
| 14,147 | 6,2554 | 267 | 10,9 |
| 14,72 | 6,01306 | 201 | 8,2 |
| 15,813 | 5,59984 | 1474 | 59,9 |
| 17,684 | 5,01117 | 223 | 9,1 |
| 18,403 | 4,81697 | 234 | 9,5 |
| 19,778 | 4,4851 | 118 | 4,8 |
| 21,31 | 4,16604 | 145 | 5,9 |
| 23,051 | 3,8552 | 148 | 6 |
| 23,874 | 3,72413 | 840 | 34,1 |
| 24,184 | 3,67713 | 702 | 28,5 |
| 24,963 | 3,56407 | 360 | 14,6 |

(suite)

| ANGLE 2 (°) | d (Angstrom) | Intensité (count) | Intensité (%) |
|---|---|---|---|
| 25,179 | 3,53403 | 395 | 16,1 |
| 25,731 | 3,4594 | 248 | 10,1 |
| 26,28 | 3,38832 | 589 | 23,9 |
| 26,693 | 3,33691 | 731 | 29,7 |
| 27,218 | 3,27369 | 431 | 17,5 |
| 27,872 | 3,19828 | 468 | 19 |
| 28,62 | 3,1164 | 251 | 10,2 |
| 29,469 | 3,02855 | 129 | 5,2 |

**Exemple 6 : préparation de la 5-hexyloxy-dinaphto[1,2-b:2',3'-d]furan-7,12-dione**

Formule :

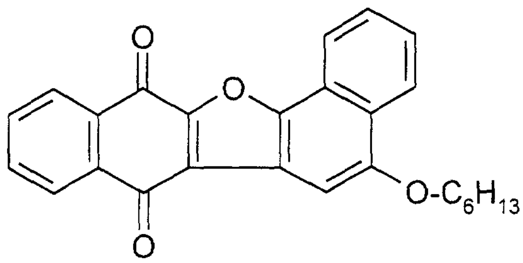

Dans un tricol de 1 litre, on solubilise 28,4 g (0,116 mol) de 2,3-dichloro-[1,4]naphtoquinone et 26,4 g (0,116 mol) de 4-hexyloxy-naphthalèn-1-ol dans 340 ml de pyridine. La solution est portée à 75°C sous agitation pendant 16 heures. On refroidit à 5°C et on filtre le solide sur verre fritté. On lave les cristaux à l'eau puis au méthanol et à l'éthanol, et on sèche.
On obtient 27,0 g (rendement : 58%) de 5-hexyloxy-dinaphto[1,2-b:2',3'-d]furan-7,12-dione sous forme de cristaux rouge orangé.

- point de fusion : 173,9°C (DSC)
- HPCCM : monospot
- RMN $^1H$ ($CDCl_3$) : conforme
- Analyse élémentaire :

| | C | H | O |
|---|---|---|---|
| Théorique | 78,38 | 5,57 | 16,06 |
| expérimental | 78,13 | 5,67 | 16,01 |

- spectre UV-Visible : $CHCl_3$: $\lambda_{max}$ nm (ε) : 485,5(50500), 266,5(378900), 235,5 (198600), 223,0(197000), 206,0 (182700).

**Exemple 7 : préparation de la 5-[cholest-5-en-3β-ol]-dinaphto[1,2-b:2',3'-d] furan-7,12-dione**

Formule :

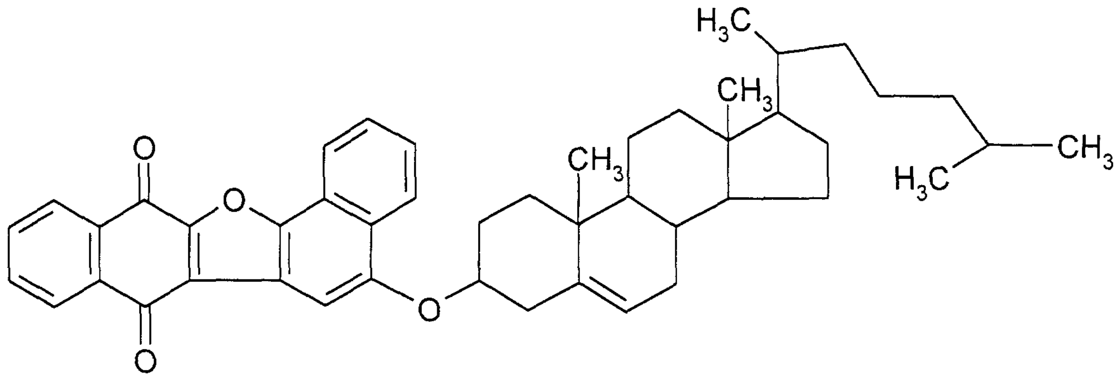

Dans un tricol de 100 ml, on solubilise 0,602 g (2,65 mmol) de 2,3-dichloro-[1,4]naphtoquinone et 1,40 g (2,65 mmol) de 4-[(3β)-cholest-5-en-3-oxy]-naphthalèn-1-ol dans 15 ml de pyridine. La solution est portée à 85°C sous agitation pendant 17 heures. On refroidit à 5°C et on filtre le solide sur verre fritté. On lave les cristaux à l'eau puis au méthanol et à l'heptane, puis on sèche.
On obtient 0,89 g (rendement : 49%) de 5-[(3β)-cholest-5-en-3-ol]-dinaphto[1,2-b:2',3'-d]furan-7,12-dione sous forme de cristaux rouge vif.

- point de fusion : 285,6°C (DSC)
- HPCCM : monospot
- RMN $^1$H ($CDCl_3$) : conforme
- spectre de masse : conforme

**Exemple 8 : préparation de la (dinaphto[2,1-b:2'.3'-d]furan-8,13-dione)[3,4-b]naphto[2',3'-d]furan-5,14-dione**

Formule :

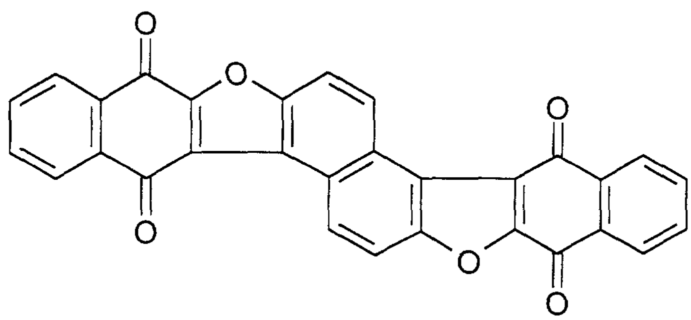

Dans un tricol de 100 ml, on solubilise 4,54 g (20 mmol) de 2,3-dichloro-[1,4]naphtoquinone et 1,60 g (10 mmol) de naphtalène-2,6-diol dans 55 ml de pyridine. La solution est portée à 80°C sous agitation pendant 18 heures, puis on refroidit à 5°C et filtre le solide sur verre fritté. On lave les cristaux au méthanol puis au dichlorométhane et l'on sèche.
On obtient 4,66 g (rendement : 99%) de composé recherché, sous forme de cristaux marron orangé insolubles.

- HPCCM : monospot
- spectre de masse : conforme

**Exemple 9 : préparation de la naphto[2,3-b]-5-aza-phenanthro[3',4'-d]furan-10,15-dione**

Formule :

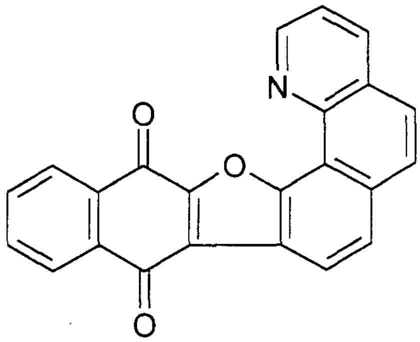

Dans un tricol de 100 ml, on solubilise 2,27 g (0,010 mol) de 2,3-dichloro-[1,4]naphtoquinone et 1,95 g (0,010 mol) de benzo[h]quinolin-10-ol dans 35 ml de pyridine. La solution est portée à 80°C sous agitation pendant 18 heures. Puis, on refroidit à 0°C et on extrait à l'acétate d'éthyle un solide qui est purifié sur colonne de silice (Eluant: $CH_2Cl_2$ 9 / AcOEt 1). On isole finalement 0,20 g (rendement : 5%) de composé recherché sous forme de cristaux orange.

- point de fusion : supérieur à 350°C (DSC)
- HPCCM : monospot
- RMN $^1H$ ($CDCl_3$) : conforme

**Exemple 10**

On a mesuré la force colorante, la couvrance, la stabilité photochimique et le dégorgement de certains composés selon l'invention.

1/ Force colorante

On obtient les valeurs de L, a, et b suivantes :

| Composé | L | a | b | C |
|---|---|---|---|---|
| 5-chloro-dinaphto[1,2-b:2',3'-d]furan-7,12-dione | 85,7 | -2,7 | 103 | 103 |
| 5-éthoxy-dinaphto[1,2-b:2',3'-d]furan-7,12-dione | 35,0 | 44,4 | 28,6 | 52,8 |
| 5-isopropyloxy-dinaphto[1,2-b:2',3'-d]furan-7,12-dione | 44,9 | 54,9 | 47,3 | 72,4 |
| dinaphto[2,1-b:2',3'-d]furan-8,13-dione | 75,6 | 10,7 | 88,0 | 88,7 |
| 2-méthoxy-dinaphto[2,1-b:2',3'-d]furan-8,13-dione | 54,5 | 53,0 | 58,4 | 78,8 |
| 3-bromo-dinaphto[2,1-b:2',3'-d]furan-8,13-dione | 82,5 | 7,6 | 99,1 | 99,4 |
| 3-méthoxy-dinaphto[2,1-b:2',3'-d]furan-8,13-dione | 70,7 | 34,2 | 85,8 | 92,4 |
| 5-hexyloxy-dinaphto[1,2-b:2',3'-d]furan-7,12-dione | 48,9 | 50,8 | 51,0 | 72,0 |
| 2-hydroxy-dinaphto[1,2-b:2',3'-d]furan-7,12-dione | 47,4 | 43,9 | 45,0 | 62,9 |
| dinaphto[1,2-b:2',3'-d]furan-7,12-dione | 88,5 | 0,19 | 108 | 108 |
| 5-hydroxy-dinaphto[2,1-b:2',3'-d]furan-8,13-dione | 22,4 | 16,9 | 9,5 | 19,4 |

Ces composés présentent donc des couleurs pures et saturées, ainsi qu'une force colorante au moins comparable à celles des pigments usuels.

2/ Couvrance

- pour la 5-hydroxy-dinaphto[2,1-b:2',3'-d]furan-8,13-dione: $\Delta E^* = 11,1$

Le composé est donc couvrant.

3/ Stabilité photochimique

- pour la 5-chloro-dinaphto[1,2-b:2',3'-d]furan-7,12-dione ΔE = 1,36
- pour la 5-éthoxy-dinaphto[1,2-b:2',3'-d]furan-7,12-dione ΔE = 0,24
- pour la 5-isopropyloxy-dinaphto[1,2-b:2',3'-d]furan-7,12-dione ΔE = 0,81
- pour la dinaphto[2,1-b:2',3'-d]furan-8,13-dione ΔE = 0,23
- pour la 2-méthoxy-dinaphto[2,1-b:2',3'-d]furan-8,13-dione ΔE = 0,28
- pour la 3-bromo-dinaphto[2,1-b:2',3'-d]furan-8,13-dione ΔE = 2,61
- pour la 3-méthoxy-dinaphto[2,1-b:2',3'-d]furan-8,13-dione ΔE = 0,81
- pour la 5-hexyloxy-dinaphto[1,2-b:2',3'-d]furan-7,12-dione ΔE = 0,91
- pour la 2-hydroxy-dinaphto[1,2-b:2',3'-d]furan-7,12-dione ΔE = 1,32
- pour la dinaphto[1,2-b:2',3'-d]furan-7,12-dione ΔE = 3,91
- pour la 5-hydroxy-dinaphto[2,1-b:2',3'-d]furan-8,13-dione ΔE = 0,10

Les composés sont donc stables photochimiquement.

4/ Dégorgement

a/ dans l'eau

- pour la 5-chloro-dinaphto[1,2-b:2',3'-d]furan-7,12-dione D.O. = 0,02
- pour la 5-éthoxy-dinaphto[1,2-b:2',3'-d]furan-7,12-dione D.O. = 0,27
- pour la 5-isopropyloxy-dinaphto[1,2-b:2',3'-d]furan-7,12-dione D.O. = 0,03
- pour la dinaphto[2,1-b:2',3'-d]furan-8,13-dione D.O. = 0,01
- pour la 2-méthoxy-dinaphto[2,1-b:2',3'-d]furan-8,13-dione D.O. = 0,30
- pour la 3-bromo-dinaphto[2,1-b:2',3'-d]furan-8,13-dione D.O. = 0,03
- pour la 3-méthoxy-dinaphto[2,1-b:2',3'-d]furan-8,13-dione D.O. = 0,04
- pour la 5-hexyloxy-dinaphto[1,2-b:2',3'-d]furan-7,12-dione D.O. = 0,29
- pour la 2-hydroxy-dinaphto[1,2-b:2',3'-d]furan-7,12-dione D.O. = 0,23
- pour la dinaphto[1,2-b:2',3'-d]furan-7,12-dione D.O. = 0,13
- pour la 5-hydroxy-dinaphto[2,1-b:2',3'-d]furan-8,13-dione D.O. = 0,16

b/ dans l'huile (triglycérides)

- pour la 3-bromo-dinaphto[2,1-b:2',3'-d]furan-8,13-dione D.O. = 0,92
- pour la 3-méthoxy-dinaphto[2,1-b:2',3'-d]furan-8,13-dione D.O. = 0,88

Les composés dégorgent donc peu dans l'eau et l'huile.

**Exemple 11**

On a comparé la force colorante, la stabilité photochimique et le dégorgement de composés selon l'invention en comparaison avec des pigments de l'art antérieur.

1/ Force colorante

| Composé | L | a | b | C |
|---|---|---|---|---|
| 3-bromo-dinaphto[2,1-b:2',3'-d]furan-8,13-dione | 82,5 | 7,6 | 99,1 | 99,4 |
| FD&C Yellow n°5 | 83,5 | 16,1 | 98,8 | 100 |
| 3-méthoxy-dinaphto[2,1-b:2',3'-d]furan-8,13-dione | 70,7 | 34,2 | 85,8 | 92,4 |
| FD&C Yellow n°6 | 58,2 | 51,4 | 73,3 | 89,5 |

Les composés selon l'invention présentent donc une couleur au moins aussi, voire plus, saturée et une force colorante au moins comparable à celle d'un pigment usuel de même teinte.

2/ Stabilité photochimique

- pour la 3-bromo-dinaphto[2,1-b:2',3'-d]furan-8,13-dione (jaune) ΔE = 2,61
- pour le FD&C Yellow n°5 (jaune) ΔE = 6,63
- pour la 3-méthoxy-dinaphto[2,1-b:2',3'-d]furan-8,13-dione (orange) ΔE = 0,81
- pour le FD&C Yellow n°6 (orange) ΔE = 10,06

Les composés selon l'invention sont donc plus stables photochimiquement que certains pigments usuels.

3/ Dégorgement dans l'eau

- pour la 3-bromo-dinaphto[2,1-b:2',3'-d]furan-8,13-dione D.O. = 0,03
- pour le FD&C Yellow n°5 (jaune) D.O. = 0,08
- pour la 3-méthoxy-dinaphto[2,1'-b:2',3'-d]furan-8,13-dione D.O. = 0,04
- pour le FD&C Yellow n°6 (orange) D.O. = 0,58

Les composés selon l'invention dégorgent donc moins dans l'eau que certains pigments usuels.

**Exemple 12**

On prépare une crème teintée de type émulsion huile-dans-eau comprenant :

- huile de parléam 22 g
- acide stéarique 1,5 g
- Polysorbate 60 (Tween 60 de ICI) 0,9 g
- alcool cétylique 0,5 g
- mélange monostéarate de glycérol/stéarate de PEG 100 2,1 g
- triéthanolamine 0,75 g
- composé de l'exemple 3 10 g
- propylène glycol 3 g
- cyclopentadiméthylsiloxane 3 g
- Carbopol 981 0,15 g
- gomme de xanthane 0,2 g
- eau qsp 100 g

**Exemple 13**

On prépare un fard à paupières comprenant les ingrédients suivants :

- talc 38 g
- mica 20 g
- oxychlorure de bismuth 8 g
- stéarate de zinc 3 g
- poudre de Nylon 20 g
- composé de l'exemple 1 5 g
- liant gras qsp 100 g

**Exemple 14 : Préparation du 3-Hydroxy-dinaphtho[2,1-b;2',3'-d]furan-8,13-dione**

Dans un tricol de 500ml, on solubilise à température ambiante 16.02g (0,1mol) de 7-hydroxy-naphtalèn-1-ol et 22,71g (0.1mol) de 2,3-dichloro-[1,4]naphtoquinone dans 300ml de pyridine. On porte le mélange sous agitation au reflux du solvant pendant 3h. Puis on refroidit le mélange réactionnel à 10°C pendant 30mn et filtre le solide sur verre fritté. On lave les cristaux à l'eau, l'éthanol et l'éther isopropylique et on sèche sous vide pour obtenir finalement 19.8 g (Rdt.:63%) de 3-Hydroxy-dinaphtho[2,1-b;2',3'-d]furan-8,13-dione sous forme de cristaux rouge.

| Point de fusion | 317.6°C (DSC) Monospot |
|---|---|
| HPCCM<br>RMN $^1H$ ($CDCl_3$) | Monospot<br>conforme |

| Analyse élémentaire | | | |
|---|---|---|---|
| | C | H | O |
| Théorique | 76.43 | 3.21 | 20.36 |
| Expérimental | 75.88 | 3.19 | 20.45 |

| Spectre DX : | | | |
|---|---|---|---|
| **ANGLE 2 (°)** | **d (Angstrom)** | **Intensité (count)** | **Intensité (%)** |
| 6,528 | 13,52963 | 243 | 12 |
| 6,952 | 12,70451 | 331 | 16,4 |
| 8,236 | 10,72614 | 2021 | 100 |
| 9,187 | 9,61821 | 573 | 28,4 |
| 9,572 | 9,23249 | 298 | 14,7 |
| 10,906 | 8,10589 | 644 | 31,9 |
| 11,711 | 7,55029 | 278 | 13,8 |
| 12,086 | 7,3167 | 559 | 27,7 |
| 12,53 | 7,05882 | 439 | 21,7 |
| 14,525 | 6,09312 | 266 | 13,2 |
| 15,084 | 5,86885 | 332 | 16,4 |
| 15,676 | 5,64831 | 367 | 18,2 |
| 17,033 | 5,20123 | 362 | 17,9 |
| 17,909 | 4,94866 | 289 | 14,3 |
| 18,687 | 4,74446 | 205 | 10,1 |
| 19,048 | 4,65538 | 255 | 12,6 |
| 20,72 | 4,28332 | 181 | 9 |

(suite)

| Spectre DX : | | | |
|---|---|---|---|
| **ANGLE 2 (°)** | **d (Angstrom)** | **Intensité (count)** | **Intensité (%)** |
| 21,244 | 4,1788 | 236 | 11,7 |
| 21,736 | 4,08539 | 182 | 9 |
| 22,719 | 3,91078 | 351 | 17,4 |
| 23,676 | 3,75484 | 261 | 12,9 |
| 24,306 | 3,65894 | 261 | 12,9 |
| 25,346 | 3,51103 | 541 | 26,8 |
| 25,956 | 3,42994 | 246 | 13,7 |
| 26,799 | 3,32394 | 563 | 27,9 |
| 27,16 | 3,28054 | 405 | 20 |
| 27,881 | 3,19734 | 391 | 19,3 |
| 28,299 | 3,15109 | 375 | 18,6 |
| 29,433 | 3,0322 | 161 | 8 |

**Exemple 15 : Préparation du 2-Hydroxy-dinaphtho[1,2-b;2',3'-d]furan-7,12-dione**

Dans un tricol de 500ml, on solubilise à température ambiante 16.02g (0,1mol) de 7-hydroxy-naphtalèn-2-ol et 22,71g (0.1mol) de 2,3-dichloro-[1,4]naphtoquinone dans 300ml de pyridine. On porte le mélange sous agitation au reflux du solvant pendant 3h. Puis on refroidit le mélange réactionnel à 10°C pendant 30mn et filtre le solide sur verre fritté. On lave les cristaux à l'eau, l'éthanol et l'éther isopropylique et sèche sous vide pour obtenir finalement 16.34g (Rdt.:52%) 2-Hydroxy-dinaphtho[1,2-b;2',3'-d]furan-7,12-dione sous forme de cristaux rouge foncé.

| | |
|---|---|
| Point de fusion | 332.4°C (DSC) |
| HPCCM | Monospot |
| RMN $^1H$ ($CDCl_3$) | conforme |

| Analyse élémentaire | | | |
|---|---|---|---|
| | C | H | O |
| Théorique | 76.43 | 3.21 | 20.36 |
| Expérimental | 75.57 | 3.20 | 20.45 |

| Spectre DX | | | |
|---|---|---|---|
| **ANGLE 2θ°** | **d (Angstrom)** | **Intensité (count)** | **Intensité (%)** |
| 7,227 | 12,22148 | 2302 | 100 |
| 9,337 | 9,46393 | 397 | 17,2 |
| 11,298 | 7,82508 | 330 | 14,3 |
| 12,341 | 7,16606 | 231 | 10 |
| 14,451 | 6,12422 | 1694 | 73,6 |
| 15,747 | 5,62309 | 540 | 23,5 |
| 16,866 | 5,25228 | 279 | 12,1 |

(suite)

| Spectre DX | | | |
|---|---|---|---|
| **ANGLE 2θ°** | **d (Angstrom)** | **Intensité (count)** | **Intensité (%)** |
| 17,45 | 5,07802 | 195 | 8,5 |
| 18,023 | 4,91781 | 218 | 9,5 |
| 18,748 | 4,72927 | 165 | 7,2 |
| 19,558 | 4,53501 | 273 | 11,9 |
| 20,862 | 4,25446 | 238 | 10,3 |
| 21,808 | 4,07194 | 152 | 6,6 |
| 22,785 | 3,89965 | 1058 | 46 |
| 23,575 | 3,77064 | 267 | 11,6 |
| 24,725 | 3,59788 | 178 | 7,7 |
| 25,276 | 3,52067 | 281 | 12,2 |
| 26,065 | 3,41582 | 118 | 5,1 |
| 26,873 | 3,31496 | 1287 | 55,9 |
| 28,836 | 3,09358 | 721 | 31,3 |
| 29,343 | 3,04127 | 132 | 5,7 |

**Revendications**

**1.** Composition cosmétique comprenant, dans un milieu cosmétiquement acceptable, au moins un composé de formule (I) :

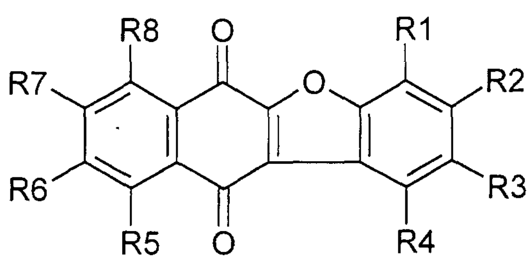

dans laquelle les radicaux R1 à R8 représentent, indépendamment les uns des autres :

- un atome d'hydrogène;
- un atome d'halogène (chlore, brome, iode, fluor);
- un radical hydroxy (-OH);
- un radical amino -NRR' avec R et R', indépendamment l'un de l'autre, représentent H ou un radical hydrocarboné, linéaire, ramifié ou cyclique, saturé ou insaturé, ayant 1 à 36 atomes de carbone;
- un radical nitro ($-NO_2$);
- un radical alkylamido -NH-CO-R" avec R" représentant un radical hydrocarboné, linéaire, ramifié ou cyclique, saturé ou insaturé, ayant 1 à 36 atomes de carbone;
- un radical uréido -NH-CO-NH-R"' avec R"' représentant H ou un radical hydrocarboné, linéaire, ramifié ou cyclique, saturé ou insaturé, ayant 1 à 36 atomes de carbone;
- un radical alkyluréthanne de formule -O-CO-NHR"" avec R"" représentant un radical hydrocarboné, linéaire, ramifié ou cyclique, saturé ou insaturé, ayant 1 à 36 atomes de carbone;
- un radical dialkylsiloxane de formule :

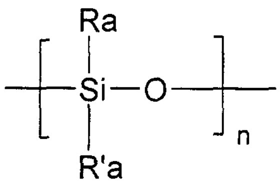

dans laquelle

- n est un entier compris entre 1 et 12;
- Ra et R'a, indépendamment l'un de l'autre, représentent un radical hydrocarboné, linéaire, ramifié ou cyclique, saturé ou insaturé, ayant 1 à 36 atomes de carbone, qui peut éventuellement comporter 1 à 12 hétéroatomes choisis parmi O, N, S et Si et/ou qui peut être substitué par un ou plusieurs substituants;

- un radical trialkylsilane de formule :

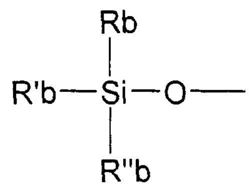

dans laquelle Rb, R'b et R"b, indépendamment l'un de l'autre, représentent un radical hydrocarboné, linéaire, ramifié ou cyclique, saturé ou insaturé, ayant 1 à 36 atomes de carbone, qui peut éventuellement comporter 1 à 12 hétéroatomes choisis parmi O, N, S et Si et/ou qui peut être substitué;
- un radical hydrocarboné, linéaire, ramifié ou cyclique, saturé ou insaturé, ayant 1 à 36 atomes de carbone, qui peut éventuellement comporter 1 à 12 hétéroatomes choisis parmi O, N, S et Si et/ou qui peut être substitué;
- les radicaux R1 et R2 ensemble et/ou R3 et R4 ensemble pouvant former, avec une chaîne hydrocarbonée, un cycle, saturé ou insaturé, comportant au total 5 ou 6 atomes de carbone, pouvant être lui-même interrompu par un ou plusieurs hétéroatomes choisis parmi O, N et S, et/ou substitué par un ou plusieurs substituants; ou
- les radicaux R2 et R3 ensemble pouvant former, avec une chaîne hydrocarbonée, un cycle, saturé ou insaturé, comportant au total 5 ou 6 atomes de carbone, pouvant être lui-même interrompu par un ou plusieurs hétéroatomes choisis parmi O, N et S, et/ou substitué par un ou plusieurs substituants;

**2.** Composition selon la revendication précédente, dans laquelle le composé répond à la formule (I) dans laquelle :

- les radicaux R1, R2, R3 et R4 représentent, indépendamment les uns des autres, un atome d'hydrogène ou un radical hydrocarboné, linéaire, ramifié ou cyclique, saturé ou insaturé, ayant 1 à 36 atomes de carbone, qui peut éventuellement comporter 1 à 8 hétéroatomes choisis parmi O ou N, et/ou qui peut être substitué;
- les radicaux R1 et R2 ensemble et/ou R3 et R4 ensemble forment, avec une chaîne hydrocarbonée, un cycle, saturé ou insaturé, comportant au total 6 atomes de carbone, pouvant être lui-même interrompu par un ou plusieurs hétéroatomes et/ou substitué par un radical hydrocarboné, linéaire, ramifié ou cyclique, saturé ou insaturé, ayant 1 à 36 atomes de carbone, qui peut éventuellement comporter lui-même 1 à 8 hétéroatomes choisis parmi O ou N, et/ou qui peut être substitué par un radical hydrocarboné, linéaire, ramifié ou cyclique, saturé ou insaturé, ayant 1 à 36 atomes de carbone; et/ou
- les radicaux R2 et R3 ensemble forment, avec une chaîne hydrocarbonée, un cycle, saturé ou insaturé, comportant au total 6 atomes de carbone, pouvant être lui-même interrompu par un ou plusieurs hétéroatomes et/ou substitué par un radical hydrocarboné, linéaire, ramifié ou cyclique, saturé ou insaturé, ayant 1 à 36 atomes de carbone, qui peut éventuellement comporter lui-même 1 à 8 hétéroatomes choisis parmi O ou N, et/ou qui peut être substitué par un radical hydrocarboné, linéaire, ramifié ou cyclique, saturé ou insaturé, ayant 1 à 36 atomes de carbone.

**3.** Composition selon l'une des revendications précédentes, dans laquelle le composé répond à l'une des formules (II) à (VI) suivantes :

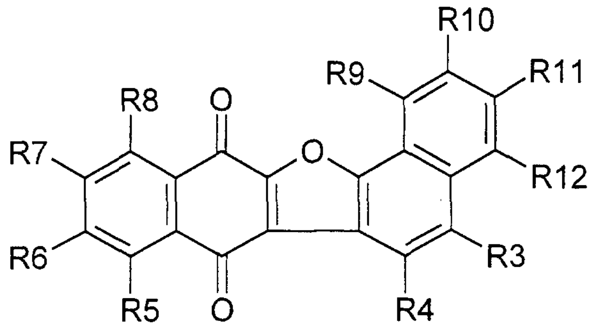

(II)

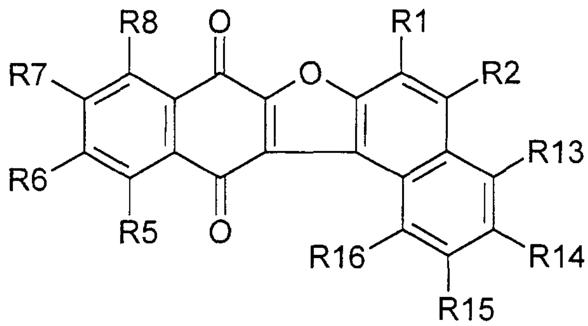

(III)

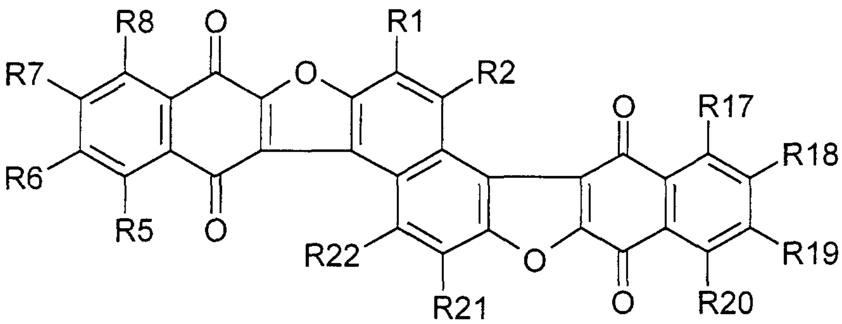

(IV)

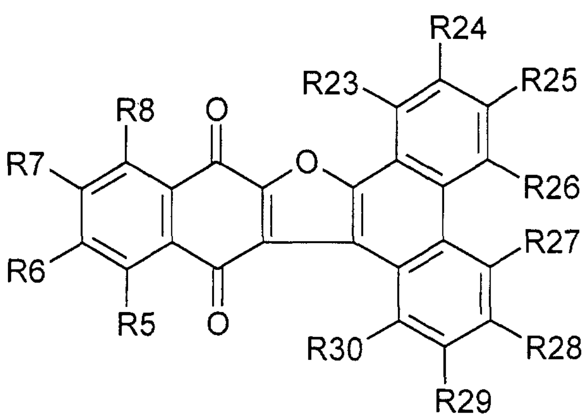

(V)

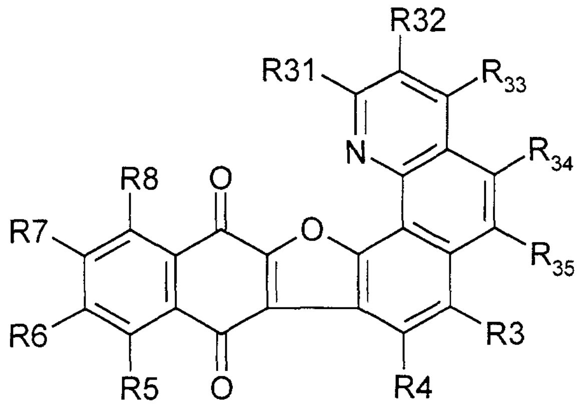

(VI)

dans lesquelles les radicaux R9 à R35 peuvent être identiques ou différents, et sont choisi parmi les significations données pour les radicaux R1 à R8 dans l'une des revendications précédentes,
étant entendu que deux radicaux adjacents peuvent former ensemble et avec une chaîne hydrocarbonée, un cycle, saturé ou insaturé, comportant au total 5 ou 6 atomes de carbone, pouvant être lui-même interrompu par un ou plusieurs hétéroatomes (O,N,S Si) et/ou substitué par un ou plusieurs substituants.

**4.** Composition selon la revendication 1, dans laquelle le composé est choisi parmi les composés de formule (I), notamment de formules (II) à (VI) et plus préférentiellement parmi les composés de formule (II) et (III), dans lesquelles les différents radicaux sont choisis, indépendamment les uns des autres, parmi :

- un atome d'hydrogène;
- un atome d'halogène (chlore, brome, iode, fluor);
- un radical hydroxy (-OH);
- un radical alcoxy (RO-) avec R en C1-12, saturé ou insaturé, linéaire ou ramifié;
- un radical alkyle, linéaire ou ramifié, saturé ou insaturé en C1-12;
- un radical acyle (R-CO-) avec R en C1-12, saturé ou insaturé, linéaire ou ramifié;
- un radical amino -NRR' avec R et R', indépendamment l'un de l'autre, représentant H ou un radical hydrocarboné, linéaire ou ramifié, saturé ou insaturé, ayant 1 à 12 atomes de carbone;
- un radical nitro (-$NO_2$).

**5.** Composition selon la revendication 1, dans laquelle le composé est choisi parmi les composés de formule (I), notamment de formules (II) à (VI) et plus préférentiellement parmi les composés de formule (II) et (III), dans lesquelles les différents radicaux sont choisis, indépendamment les uns des autres, parmi :

- un atome d'hydrogène;
- un atome de chlore ou de brome;
- un radical hydroxy (-OH);
- un radical alcoxy (RO-) avec R en C1-6, saturé ou insaturé, linéaire ou ramifié, notamment méthoxy, éthoxy ou propoxy;
- un radical alkyle, linéaire ou ramifié, saturé ou insaturé en C1-6.

**6.** Composition selon l'une des revendications précédentes, dans laquelle le composé est choisi parmi :

- la dinaphto[1,2-b:2',3'-d]furan-7,12-dione
- la 2-hydroxy-dinaphto[1,2-b:2',3'-d]furan-7,12-dione
- la 3-hydroxy-dinaphto[1,2-b:2',3'-d]furan-7,12-dione
- la 4-hydroxy-dinaphto[1,2-b:2',3'-d]furan-7,12-dione
- la 5-méthoxy-dinaphto[1,2-b:2',3'-d]furan-7,12-dione
- la 5-chloro-dinaphto[1,2-b:2',3'-d]furan-7,12-dione
- la 5-éthoxy-dinaphto[1,2-b:2',3'-d]furan-7,12-dione
- la 5-isopropyloxy-dinaphto[1,2-b:2',3'-d]furan-7,12-dione
- la 5-hexyloxy-dinaphto[1,2-b:2',3'-d]furan-7,12-dione
- la 5-[cholest-5-en-3β-ol]-dinaphto[1,2-b:2',3'-d]furan-7,12-dione

- la dinaphto[2,1-b:2',3'-d]furan-8,13-dione
- la 2-méthoxy-dinaphto[2,1-b:2',3'-d]furan-8,13-dione
- la 3-bromo-dinaphto[2,1-b:2',3'-d]furan-8,13-dione
- la 3-hydroxy-dinaphto[2,1-b:2',3'-d]furan-8,13-dione
- la 3-méthoxy-dinaphto[2,1-b:2',3'-d]furan-8,13-dione
- la 5-hydroxy-dinaphto[2,1-b:2',3'-d]furan-8,13-dione
- la (dinaphto[2,1-b:2',3'-d]furan-8,13-dione)[3,4-b]naphto[2',3'-d]furan-5,14-dione
- la naphto[2,3-b]-phénanthro[9,10-d]furan-10,15-dione
- la naphto[2,3-b]-5-azo-phenanthro[3',4'-d]furan-10,15-dione.

**7.** Composition selon l'une des revendications précédentes, comprenant 0,01 à 50% en poids par rapport au poids total de la composition, de préférence 0,1 à 20% en poids, notamment 0,5 à 10% en poids, de composé de formule (I).

**8.** Composition selon l'une des revendications précédentes, dans laquelle le milieu cosmétiquement acceptable comprend ou se présente sous la forme d'une suspension, une dispersion, une solution en milieu solvant ou hydroalcoolique, éventuellement épaissie voire gélifiée; une émulsion huile-dans-eau, eau-dans-huile, ou multiple; un gel ou une mousse; un gel émulsionné; une dispersion de vésicules notamment lipidiques; une lotion biphase ou multiphase; un spray; une poudre libre, compacte ou coulée; une pâte anhydre.

**9.** Composition selon l'une des revendications précédentes, se présentant sous la forme d'un produit à appliquer sur les muqueuses, les semi-muqueuses et/ou les tissus kératiniques tels que la peau et les phanères (ongles, cils, sourcils, poils et cheveux).

**10.** Composition selon l'une des revendications précédentes, se présentant :

- sous la forme d'un produit de maquillage de la peau du visage, du corps ou des lèvres ou des fibres kératiniques (ongles, cils, sourcils, cheveux), tel qu'un fond de teint, une crème teintée, un fard à joues ou à paupières, une poudre libre ou compacte, un stick anti-cernes, un stick camouflant, un eye-liner, un mascara, un rouge à lèvres, un vernis-à-ongles, une composition de maquillage des cheveux;
- sous la forme d'un produit de soin de la peau du visage ou du corps y compris le cuir chevelu, tel qu'une composition de soin (de jour, de nuit, anti-rides, hydratante, etc.) pour le visage; une composition matifiante pour le visage;
- sous la forme d'une composition de protection solaire ou de bronzage artificiel (autobronzant);
- sous la forme d'une composition capillaire, et notamment une crème ou un gel coiffant, une composition de teinture, d'oxydation ou directe, éventuellement sous forme de shampooing colorant;

**11.** Utilisation d'au moins un composé de formule (I) tel que défini dans la revendication 1, en tant qu'agent colorant, notamment dans une composition cosmétique.

**12.** Utilisation selon la revendication 11, dans une composition cosmétique se présentant sous la forme d'un produit à appliquer sur les muqueuses, les semi-muqueuses et/ou les tissus kératiniques tels que la peau et les phanères (ongles, cils, sourcils, poils et cheveux).

**13.** Utilisation selon l'une des revendications 11 à 12, dans une composition cosmétique se présentant :

- sous la forme d'un produit de maquillage de la peau du visage, du corps ou des lèvres ou des fibres kératiniques (ongles, cils, sourcils, cheveux), tel qu'un fond de teint, une crème teintée, un fard à joues ou à paupières, une poudre libre ou compacte, un stick anti-cernes, un stick camouflant, un eye-liner, un mascara, un rouge à lèvres, un vernis-à-ongles, une composition de maquillage des cheveux;
- sous la forme d'un produit de soin de la peau du visage ou du corps y compris le cuir chevelu, tel qu'une composition de soin (de jour, de nuit, anti-rides, hydratante, etc.) pour le visage; une composition matifiante pour le visage;
- sous la forme d'une composition de protection solaire ou de bronzage artificiel (autobronzant);
- sous la forme d'une composition capillaire, et notamment une crème ou un gel coiffant, une composition de teinture, d'oxydation ou directe, éventuellement sous forme de shampooing colorant.

**14.** Composé dérivé de furane-naphtoquinone, répondant à l'une des formules (IIa) à (Via) suivantes :

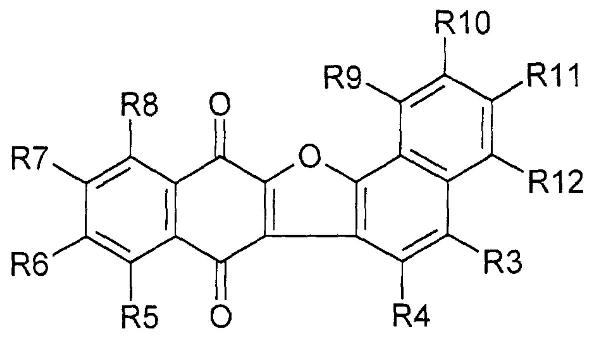

(IIa)

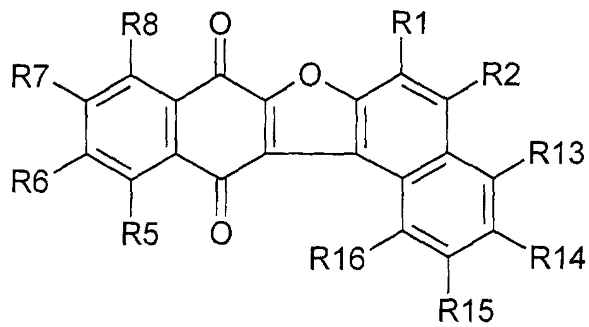

(IIIa)

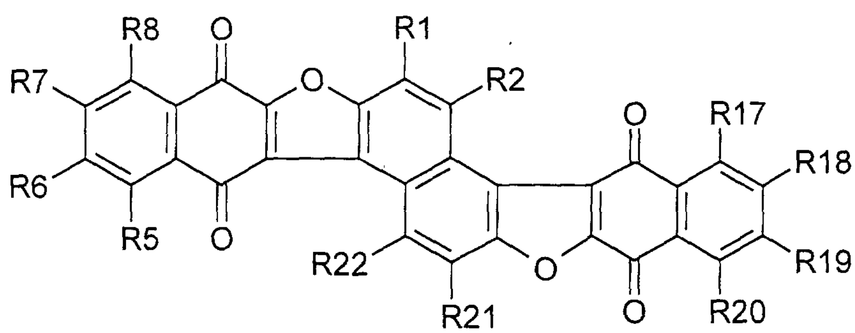

(IVa)

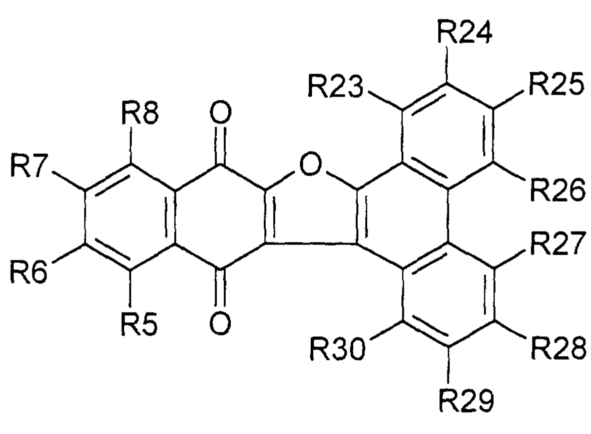

(Va)

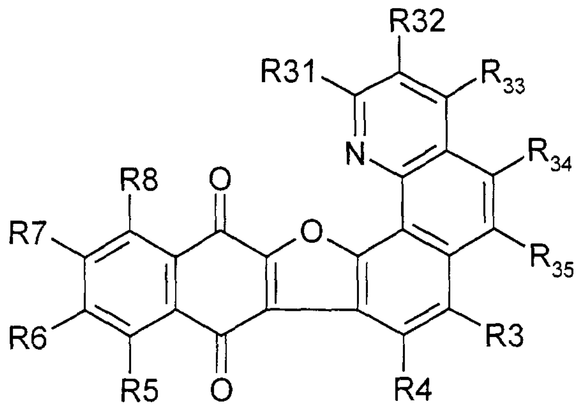

(VIa)

dans lesquelles les radicaux R9 à R35 peuvent être identiques ou différents, et sont choisi parmi les significations données pour les radicaux R1 à R8 dans l'une des revendications 1 à 2, à l'exception des composés :

- de formule (IIa) ou (IIIa) ou (Va) dans lesquelles tous les radicaux représentent H;
- de formule (IIa) dans laquelle R3 = OH et tous les autres radicaux représentent H;
- de formule (IIa) dans laquelle R3 = $OCH_3$ et tous les autres radicaux représentent H;
- de formule (IIa) dans laquelle R10 = OH et tous les autres radicaux représentent H;
- de formule (IIa) dans laquelle R11 = OH et tous les autres radicaux représentent H;
- de formule (IIIa) dans laquelle tous les radicaux représentent H;
- de formule (IIIa) dans laquelle R13 = $OCH_3$ et tous les autres radicaux représentent H;
- de formule (IIIa) dans laquelle R13 = OH et tous les autres radicaux représentent H;
- de formule (IIIa) dans laquelle $R_1$=OH et tous les autres radicaux représentent H;
- de formule (IIIa) dans laquelle R2=OH et tous les autres radicaux représentent H;
- de formule (IIIa) dans laquelle R1=$OCH_3$ et tous les autres radicaux représentent H;
- de formule (IIa) ou (IIIa) dans lesquelles $R_5$= $NO_2$ et tous les autres radicaux représentent H;
- de formule (IIa) ou (IIIa) dans lesquelles $R_8$ = $NO_2$ et tous les autres radicaux représentent H;
- de formule (IIa) ou (IIIa) dans lesquelles $R_5$ = $NH_2$ et tous les autres radicaux représentent H;
- de formule (IIa) ou (IIIa) dans lesquelles $R_8$ = $NH_2$ et tous les autres radicaux représentent H;
- de formule (IIa) ou (IIIa) dans lesquelles $R_5$ désigne benzamido et tous les autres radicaux représentent H;
- de formule (IIa) ou (IIIa) dans lesquelles $R_8$ désigne benzamido et tous les autres radicaux représentent H;
- de formule (IIIa) dans laquelle $R_8$= Br et tous les autres radicaux représentent H;
- de formule (IIIa) dans laquelle $R_8$= p-tolylsulfonamido et tous les autres radicaux représentent H;
- de formule (IIIa) dans laquelle $R_1$ =$OCH_3$ et tous les autres radicaux représentent H;
- de formule (IIIa) dans laquelle $R_2$ = $O(CH_2)_2$ - $N(CH_3)_2H.Cl$ et tous les autres radicaux représentent H;
- de formule (IIIa) dans laquelle $R_2$ = $O(CH_2)_3$ - $N(CH_3)_2H.Cl$ et tous les autres radicaux représentent H;
- de formules (IIIa) dans lesquelles $R_1$ est choisi parmi -CONH-(2'- pyridyl),-CONH-(2'- pyrimidinyl), -CONH-(2'- thiazolyl), -CONH-(3'(H-1,2,4-triazolyl) et-CONH-phényl et tous les autres radicaux représentent H;
- de formules (IIIa) dans lesquelles $R_1$ est choisi parmi -CONH-(2'- méthylphényl), -CONH-(4'- cyanophényl), -CONH-(2'(3'-méthoxypyridyl) et -CONH-(4'-méthoxyphényl), R14 et R15 désignent ensemble -CH=CH-CH=CH- et tous les autres radicaux représentent H;
- de formules (IIIa) dans lesquelles $R_1$ a la formule suivante :

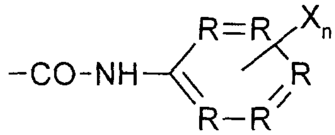

dans laquelle R est choisi parmi N et CH, de 1 à 3 radicaux R désignent N, X est choisi parmi H, $CH_3$, $C_2H_5$, $NO_2$, $OCH_3$, CN, $SO_2NH_2$, $CO_2CH_3$, $CO_2C_2H_5$, $SO_2NHC_6H_5$, Cl, F, Br et I,
n est un entier positif de 1 à 4.

**15.** Composé selon la revendication 14, **caractérisé en ce qu'**il est choisi parmi :

- la '5-chloro-dinaphto[1,2-b:2',3'-d]furan-7,12-dione,
- la 5-isopropyloxy-dinaphto[1,2-b:2',3'-d]furan-7,12-dione,
- la 2-méthoxy-dinaphto[2,1-b:2',3'-d]furan-8,13-dione,
- la 3-bromo-dinaphto[2,1-b:2',3'-d]furan-8,13-dione,
- la 5-hexyloxy-dinaphto[1,2-b:2',3'-d]furan-7,12-dione,
- la 5-[cholest-5-en-3β-ol]-dinaphto[1,2-b:2',3'-d]furan-7,12-dione,
- la (dinaphto[2,1-b:2',3'-d]furan-8,13-dione)[3,4-b]naphto[2',3'-d]furan-5,14-dione,
- la 3-méthoxy-dinaphto[2,1-b:2',3'-d]furan-8,13-dione,
- la naphto[2,3-b]-5-azo-phenanthro[3',4'-d]furan-10,15-dione.

**16.** Composé selon la revendication 15, de formule :

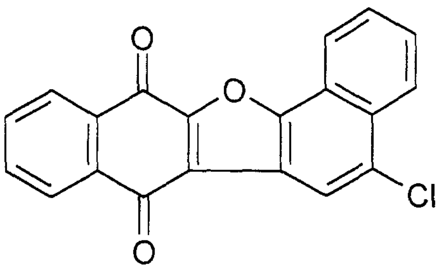

et ayant le spectre DX suivant :

| ANGLE 2 (°) | d (Angstrom) | Intensité (count) | Intensité (%) |
|---|---|---|---|
| 8,858 | 9,97422 | 1212 | 91,4 |
| 10,545 | 8,38269 | 974 | 73,5 |
| 12,759 | 6,93251 | 1326 | 100 |
| 14,369 | 6,15885 | 366 | 27,6 |
| 15,21 | 5,8203 | 194 | 14,6 |
| 17,439 | 5,08119 | 159 | 12 |
| 17,822 | 4,97271 | 143 | 10,8 |
| 19,769 | 4,48716 | 252 | 19 |
| 21,228 | 4,18188 | 173 | 13 |
| 21,813 | 4,07116 | 126 | 9,5 |
| 22,346 | 3,97513 | 295 | 22,2 |
| 24,67 | 3,60578 | 247 | 18,6 |
| 24,927 | 3,56909 | 209 | 15,8 |
| 25,992 | 3,42522 | 858 | 64,7 |
| 26,525 | 3,3576 | 393 | 29,6 |
| 27,151 | 3,28166 | 692 | 52,2 |
| 28,096 | 3,17331 | 111 | 8,4 |
| 29,412 | 3,03425 | 100 | 7,5 |

**17.** Composé selon la revendication 15 de formule :

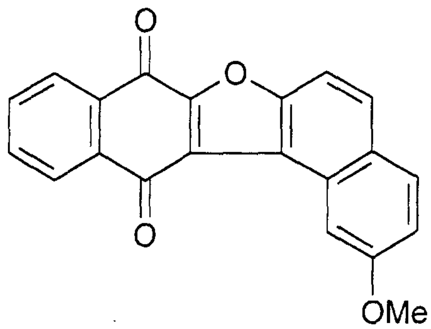

et ayant le spectre DX suivant :

| ANGLE 2 (°) | d (Angstrom) | Intensité (count) | Intensité (%) |
|---|---|---|---|
| 7,124 | 12,39813 | 1065 | 69,8 |
| 10,087 | 8,76195 | 1055 | 69,1 |
| 10,694 | 8,26606 | 1526 | 100 |
| 12,37 | 7,14936 | 890 | 58,3 |
| 13,022 | 6,79302 | 273 | 17,9 |
| 14,341 | 6,17099 | 453 | 29,7 |
| 14,784 | 5,98695 | 572 | 37,5 |
| 16,572 | 5,34479 | 214 | 14 |
| 16,696 | 5,30553 | 221 | 14,5 |
| 17,8 | 4,97885 | 198 | 13 |
| 18,612 | 4,76353 | 166 | 10,9 |
| 19,125 | 4,63688 | 304 | 19,9 |
| 19,56 | 4,53465 | 251 | 16,4 |
| 20,39 | 4,35189 | 223 | 14,6 |
| 20,653 | 4,2971 | 309 | 20,2 |
| 22,245 | 3,99296 | 337 | 22,1 |
| 23,524 | 3,77866 | 132 | 8,7 |
| 24,553 | 3,62271 | 168 | 11 |
| 25,317 | 3,51502 | 960 | 56,4 |
| 25,901 | 3,4371 | 1255 | 82,2 |
| 26,2 | 3,39852 | 819 | 53,7 |
| 27,568 | 3,23294 | 393 | 25,8 |
| 27,678 | 3,22028 | 420 | 27,5 |
| 29,622 | 3,01328 | 178 | 11,7 |

**18.** Composé selon la revendication 15 de formule :

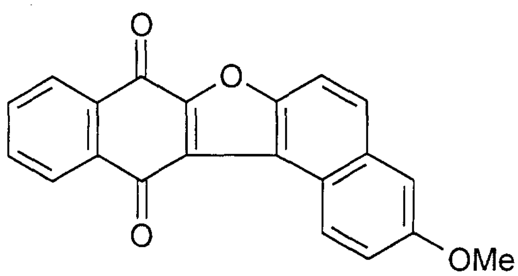

et ayant le spectre DX suivant :

| ANGLE 2 (°) | d (Angstrom) | Intensité (count) | Intensité (%) |
|---|---|---|---|
| 7,02 | 12,5816 | 1800 | 73,2 |
| 10,555 | 8,3741 | 621 | 25,2 |
| 11,834 | 7,47227 | 2460 | 100 |
| 12,213 | 7,24081 | 976 | 39,7 |
| 12,926 | 6,89616 | 1812 | 73,7 |
| 14,147 | 6,2554 | 267 | 10,9 |
| 14,72 | 6,01306 | 201 | 8,2 |
| 15,813 | 5,59984 | 1474 | 59,9 |
| 17,684 | 5,01117 | 223 | 9,1 |
| 18,403 | 4,81697 | 234 | 9,5 |
| 19,778 | 4,4851 | 118 | 4,8 |
| 21,31 | 4,16604 | 145 | 5,9 |
| 23,051 | 3,8552 | 148 | 6 |
| 23,874 | 3,72413 | 840 | 34,1 |
| 24,184 | 3,67713 | 702 | 28,5 |
| 24,963 | 3,56407 | 360 | 14,6 |
| 25,179 | 3,53403 | 395 | 16,1 |
| 25,731 | 3,4594 | 248 | 10,1 |
| 26,28 | 3,38832 | 589 | 23,9 |
| 26,693 | 3,33691 | 731 | 29,7 |
| 27,218 | 3,27369 | 431 | 17,5 |
| 27,872 | 3,19828 | 468 | 19 |
| 28,62 | 3,1164 | 251 | 10,2 |
| 29,469 | 3,02855 | 129 | 5,2 |